# EUROPÄISCHE PATENTSCHRIFT

(11) **EP 1 061 113 B1**
(45) Veröffentlichungstag und Bekanntmachung des Hinweises auf die Patenterteilung: **16.04.2003**
(21) Anmeldenummer: 00111045.1
(22) Anmeldetag: 31.05.2000
(51) Int. Cl.: C09K 19/30, C09K 19/42, C07C 43/184

(54) **Flüssigkristallines Medium**
Liquid crystal medium
Milieu liquide cristallin

(30) Priorität: 18.06.1999 DE 19927993; 18.12.1999 DE 19961305
(43) Veröffentlichungstag der Anmeldung: 20.12.2000
(73) Patentinhaber: MERCK PATENT GmbH, 64293 Darmstadt (DE)
(72) Erfinder: Bremer, Matthias, Dr., 64295 Darmstadt (DE); Lüssem, Georg, Dr., 64372 Ober-Ramstadt (DE); Klement, Dagmar, 64846 GrossZimmern (DE)

(56) Entgegenhaltungen:
- EP-A- 0 030 761
- EP-A- 0 474 062
- WO-A-00/37586
- WO-A-91/03450
- GB-A- 2 270 913
- PATENT ABSTRACTS OF JAPAN vol. 006, no. 135 (C-115), 22. Juli 1982 (1982-07-22) & JP 57 059851 A (CHISSO CORP), 10. April 1982 (1982-04-10)

## Beschreibung

Die vorliegende Erfindung betrifft ein flüssigkristallines Medium, sowie dessen Verwendung für elektrooptische Zwecke und dieses Medium enthaltende Anzeigen.

Flüssige Kristalle werden vor allem als Dielektrika in Anzeigevorrichtungen verwendet, da die optischen Eigenschaften solcher Substanzen durch eine angelegte Spannung beeinflußt werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation ausgerichteter Phasen), Gast/Wirt-Zellen, TN-Zellen mit verdrillt nematischer ("twisted nematic") Struktur, STN-Zellen ("supertwisted nematic"), SBE-Zellen ("superbirefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben.

Weiterhin sollten sie bei üblichen Betriebstemperaturen, d.h. in einem möglichst breiten Bereich unterhalb und oberhalb Raumtemperatur eine geeignete Mesophase besitzen, beispielsweise für die oben genannten Zellen eine nematische oder cholesterische Mesophase. Da Flüssigkristalle in der Regel als Mischungen mehrerer Komponenten zur Anwendung gelangen, ist es wichtig, daß die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine geringe elektrische Leitfähigkeit aufweisen.

Beispielsweise sind für Matrix-Flüssigkristallanzeigen mit integrierten nicht-linearen Elementen zur Schaltung einzelner Bildpunkte (MFK-Anzeigen) Medien mit großer positiver dielektrischer Anisotropie, breiten nematischen Phasen, relativ niedriger Doppelbrechung, sehr hohem spezifischen Widerstand, guter UV- und Temperaturstabilität und geringem Dampfdruck erwünscht.

Derartige Matrix-Flüssigkristallanzeigen sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei man zwei Typen unterscheiden kann:
1. MOS (Metal Oxide Semiconductor) oder andere Dioden auf Silizium-Wafer als Substrat.
2. Dünnfilm-Transistoren (TFT) auf einer Glasplatte als Substrat.

Die Verwendung von einkristallinem Silizium als Substratmaterial beschränkt die Displaygröße, da auch die modulartige Zusammensetzung verschiedener Teildisplays an den Stößen zu Problemen führt.

Bei dem aussichtsreicheren Typ 2, welcher bevorzugt ist, wird als elektrooptischer Effekt üblicherweise der TN-Effekt verwendet. Man unterscheidet zwei Technologien: TFTs aus Verbindungshalbleitern wie z.B. CdSe oder TFT's auf der Basis von polykristallinem oder amorphem Silizium. An letzterer Technologie wird weltweit mit großer Intensität gearbeitet.

Die TFT-Matrix ist auf der Innenseite der einen Glasplatte der Anzeige aufgebracht, während die andere Glasplatte auf der Innenseite die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, daß je ein Filterelement einem schaltbaren Bildelement gegenüber liegt.

Die TFT-Anzeigen arbeiten üblicherweise als TN-Zellen mit gekreuzten Polarisatoren in Transmission und sind von hinten beleuchtet.

Der Begriff MFK-Anzeigen umfaßt hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen (z.B. Taschenfernseher) oder für hochinformative Displays für Rechneranwendungen (Laptop) und im Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris]. Mit abnehmendem Widerstand verschlechtert sich der Kontrast einer MFK-Anzeige und es kann das Problem der "after image elimination" auftreten. Da der spezifische Widerstand der Flüssigkristallmischung durch Wechselwirkung mit den inneren Oberflächen der Anzeige im allgemeinen über die Lebenszeit einer MFK-Anzeige abnimmt, ist ein hoher (Anfangs)-Widerstand sehr wichtig, um akzeptable Standzeiten zu erhalten. Insbesondere bei low-volt-Mischungen war es bisher nicht möglich, sehr hohe spezifische Widerstände zu realisieren. Weiterhin ist es wichtig, daß der spezifische Widerstand eine möglichst geringe Zunahme bei steigender Temperatur sowie nach Temperatur- und/oder UV-Belastung zeigt. Besonders nachteilig sind auch die Tieftemperatureigenschaften der Mischungen aus dem Stand der Technik. Gefordert wird, daß auch bei tiefen Temperaturen keine Kristallisation und/oder smektische Phasen auftreten und die Temperaturabhängigkeit der Viskosität möglichst gering ist. Die MFK-Anzeigen aus dem Stand der Technik genügen somit nicht den heutigen Anforderungen.

Es besteht somit immer noch ein großer Bedarf nach MFK-Anzeigen mit sehr hohem spezifischen Widerstand bei gleichzeitig großem Arbeitstemperaturbereich, kurzen Schaltzeiten auch bei tiefen Temperaturen und niedriger Schwellenspannung, die diese Nachteile nicht oder nur in geringerem Maße zeigen.

Bei TN-(Schadt-Helfrich)-Zellen sind Medien erwünscht, die folgende Vorteile in den Zellen ermöglichen:
- erweiterter nematischer Phasenbereich (insbesondere zu tiefen Temperaturen)
- Schaltbarkeit bei extrem tiefen Temperaturen (out-door-use, Automobil, Avionik)
- erhöhte Beständigkeit gegenüber UV-Strahlung (längere Lebensdauer)

Mit den aus dem Stand der Technik zur Verfügung stehenden Medien ist es nicht möglich, diese Vorteile unter gleichzeitigem Erhalt der übrigen Parameter zu realisieren.

Bei höher verdrillten Zellen (STN) sind Medien erwünscht, die eine höhere Multiplexierbarkeit und/oder kleinere Schwellenspannungen und/oder breitere nematische Phasenbereiche (insbesondere bei tiefen Temperaturen) ermöglichen. Hierzu ist eine weitere Ausdehnung des zur Verfügung stehenden Parameterraumes (Klärpunkt, Übergang smektisch-nematisch bzw. Schmelzpunkt, Viskosität, dielektrische Größen, elastische Größen) dringend erwünscht.

Der Erfindung liegt die Aufgabe zugrunde, Medien insbesondere für derartige MFK-, TN- oder STN-Anzeigen bereitzustellen, die die oben angegebenen Nachteile nicht oder nur in geringerem Maße, und vorzugsweise gleichzeitig sehr hohe spezifische Widerstände und niedrige Schwellenspannungen aufweisen.

Es wurde nun gefunden, daß diese Aufgabe gelöst werden kann, wenn man in Anzeigen erfindungsgemäße Medien verwendet.

Gegenstand der Erfindung ist somit ein flüssigkristallines Medium auf der Basis eines Gemisches von polaren Verbindungen mit positiver dielektrischer Anisotropie, dadurch gekennzeichnet, daß es eine oder mehrere Verbindungen der allgemeinen Formel IA 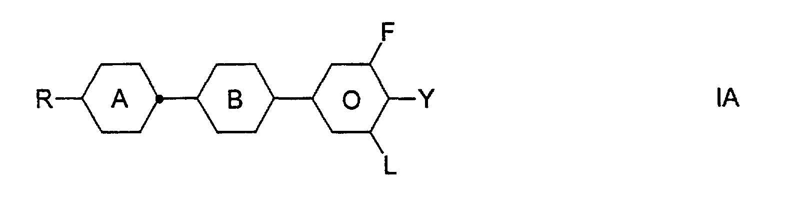 und ein oder mehrere Verbindungen der Formel IB 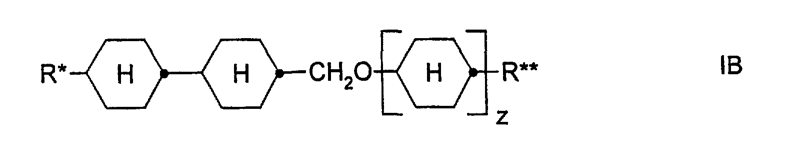 enthält,
worin
- R, R* und R**: jeweils unabhängig voneinander einen unsubstituierten, einen einfach durch CN oder CF₃ oder einen mindestens einfach durch Halogen substituierten Alkyloder Alkenylrest mit 1 bis 12 C-Atomen, wobei in diesen Resten auch eine oder mehrere CH₂-Gruppen jeweils unabhängig voneinander durch -O-, -S-, 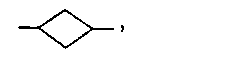 -CO-, -CO-O-, -O-CO- oder -O-CO-O- so ersetzt sein können, daß O-Atome nicht direkt miteinander verknüpft sind, 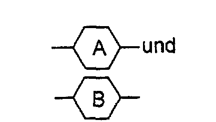 jeweils unabhängig voneinander 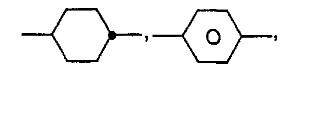 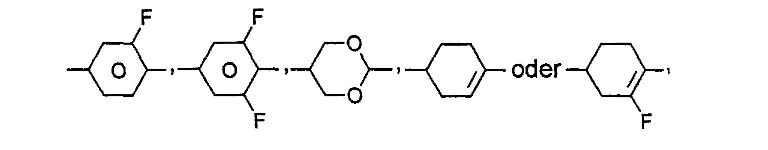
- z: 1 oder 2,
- L: H oder F, und
- Y: F, Cl, CN, halogeniertes Alkyl, halogeniertes Alkenyloxy, halogeniertes Alkoxy oder halogeniertes Alkenyl mit bis zu 9 C-Atomen
bedeuten.

Insbesondere bei low Δn-Mischungen besteht häufig das Problem, daß Verbindungen mit einem sehr hohen Klärpunkt und kleinem Δn-Wert fast immer smektische Phasen aufweisen und somit in der Mischung zu LTS-Problemen (Low Temperature Stability) führen. Durch die Verwendung der Verbindungen der Formel IB lassen sich polare Mischungen herstellen, die eine gute Reliability, eine gute LTS und einen relativ niedrigen Δn-Wert aufweisen. Weiterhin zeichnen sich die erfindungsgemäßen Mischungen durch ihre gute Holding Ratio aus.

Die Verbindungen der Formeln IA und IB besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formeln IA und IB flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Rotationsviskosität γ₁ zu optimieren ohne einen Verlust von Δn.

Die Verbindungen der Formeln IA und IB sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Die Verbindungen der Formeln IA und IB werden umfaßt von der U.S. 4,361,494 und WO91/03450. Gegenstand der Erfindung sind auch Verbindungen der Formel IB.

Falls R, R* oder R** einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3-oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R, R* oder R** einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl. Vinyl-, 1 E-Alkenyl- und 3E-Alkenylreste sind dabei bevorzugt.

Falls R, R* oder R** einen Alkylrest bedeutet, in dem eine CH₂-Gruppe durch -O- und eine durch -CO- ersetzt ist, so sind diese bevorzugt benachbart. Somit beinhalten diese eine Acyloxygruppe -CO-O- oder eine Oxycarbonylgruppe -O-CO-. Vorzugsweise sind diese geradkettig und haben 2 bis 6 C-Atome. Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R, R* oder R** einen Alkylrest bedeutet, in dem eine CH₂-Gruppe durch unsubstituiertes oder substituiertes -CH=CH- und eine benachbarte CH₂-Gruppe durch CO oder CO-O oder O-CO ersetzt ist, so kann dieser geradkettig oder verzweigt sein. Vorzugsweise ist er geradkettig und hat 4 bis 12 C-Atome. Er bedeutet demnach besonders Acryloyloxy-methyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyl-oxypentyl, 6-Methacryloyloxyhexyl, 7-Methacryloyloxyheptyl, 8-Methacryloyloxyoctyl, 9-Methacryloyloxynonyl.

Falls R, R* oder R** einen einfach durch CN oder CF₃ substituierten Alkyloder Alkenylrest bedeutet, so ist dieser Rest vorzugsweise geradkettig. Die Substitution durch CN oder CF₃ ist in beliebiger Position.

Falls R, R* oder R** einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen der Formeln IA bzw. IB, die über für Polymerisationsreaktionen geeignete Flügelgruppen R, R*, R** verfügen, eignen sich zur Darstellung flüssigkristalliner Polymerer.

Verbindungen der Formeln IA bzw. IB mit verzweigten Flügelgruppen R, R*, R** können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verbindungen der Formel IA und/oder IB mit S_{A}-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R, R*, R** sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R, R* oder R** einen Alkylrest darstellt, in dem zwei oder mehr CH₂-Gruppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxycarbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-pentyl.

In der Verbindung IA bedeutet Y vorzugsweise F, Cl, CN, CF₃, CF₂H, OCF₃, OCF₂H, OCFHCFH₂, OCFHCF₂H, OCF₂CH₃, OCF₂CFH₂, OCF₂CF₂H, OCF₂CF₂CF₂H, OCF₂CF₂CFH₂, OCFHCF₂CF₃, OCFHCF₂CF₂H, OCFHCFHCF₃, OCH₂CF₂CF₃, OCF₂CF₂CF₃, OCF₂CFHCFH₂, OCF₂CH₂CF₂H, OCFHCF₂CFH₂, OCFHCFHCF₂H, OCFHCH₂CF₃, OCH₂CFHCF₃, OCH₂CF₂CF₂H, OCF₂CFHCH₃, OCF₂CH₂CFH₂, OCFHCF₂CH₃, OCFHCFHCFH₂, OCFHCH₂CF₃, OCH₂CF₂CFH₂, OCH₂CFHCF₂H, OCF₂CH₂CH₃, OCFHCFHCH₃, OCFHCH₂CFH₂, OCH₂CF₂CH₃, OCH₂CFHCFH₂, OCH₂CH₂CF₂H, OCHCH₂CH₃, OCH₂CFHCH₃, OCH₂CH₂CF₂H, OCClFCF₃, OCClFCClF₂, OCClFCFH₂, OCFHCCl₂F, OCClFCF₂H, OCClFCClF₂, OCF₂CClH₂, OCF₂CCl₂H, OCF₂CCl₂F, OCF₂CClFH, OCF₂CClF₂, OCF₂CF₂CClF₂, OCF₂CF₂CCl₂F, OCClFCF₂CF₃, OCClFCF₂CF₂H, OCClFCF₂CClF₂, OCClFCFHCF₃, OCClFCClFCF₃, OCCl₂CF₂CF₃, OCClHCF₂CF₃, OCClFCF₂CF₃, OCClFCClFCF₃, OCF₂CClFCFH₂, OCF₂CF₂CCl₂F, OCF₂CCl₂,CF₂H, OCF₂CH₂CClF₂, OCClFCF₂CFH₂, OCFHCF₂CCl₂F, OCClFCFHCF₂H, OCClFCClFCF₂H, OCFHCFHCClF₂, OCClFCH₂CF₃, OCFHCCl₂CF₃, OCCl₂CFHCF₃, OCH₂CClFCF₃, OCCl₂CF₂CF₂H, OCH₂CF₂CClF₂, OCF₂CClFCH₃, OCF₂CFHCCl₂H, OCF₂CCl₂CFH₂, OCF₂CH₂CCl₂F, OCClFCF₂CH₃, OCFHCF₂CCl₂H, OCClFCClFCFH₂, OCFHCFHCCl₂F, OCClFCH₂CF₃, OCFHCCl₂CF₃, OCCl₂CF₂CFH₂, OCH₂CF₂CCl₂F, OCCl₂CFHCF₂H, OCClHCClFCF₂H, OCF₂CClHCClH₂, OCF₂CH₂CCl₂H, OCClFCFHCH₃, OCF₂CClFCCl₂H, OCClFCH₂CFH₂, OCFHCCl₂CFH₂, OCCl₂CF₂CH₃, OCH₂CF₂CClH₂, OCCl₂CFHCFH₂, OCH₂CClFCFCl₂, OCH₂CH₂CF₂H, OCClHCClHCF₂H, OCH₂CCl₂CF₂H, OCClFCH₂CH₃, OCFHCH₂CCl₂H, OCClHCFHCClH₂, OCH₂CFHCCl₂H, OCCl₂CH₂CF₂H, OCH₂CCl₂CF₂H, OCH = CHF, OCH = CF₂, OCF = CHF, OCF = CF₂, insbesondere F, Cl, CN, CF₃, CF₂H, OCF₃, OCF₂H, OCFHCF₃, OCFHCFH₂, OCFHCF₂H, OCF₂CH₃, OCF₂CFH₂, OCF₂CF₂H, OCF₂CF₂CF₂H, OCF₂CF₂CFH₂, OCFHCF₂CF₃, OCFHCF₂CF₂H, OCF₂CF₂CF₃, OCF₂CHFCF₃, OCClFCF₂CF₃ oder OCH = CF₂.

Die Verbindungen der Formeln IA und IB werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Verbindungen der Formel IB lassen sich beispielsweise wie folgt herstellen:

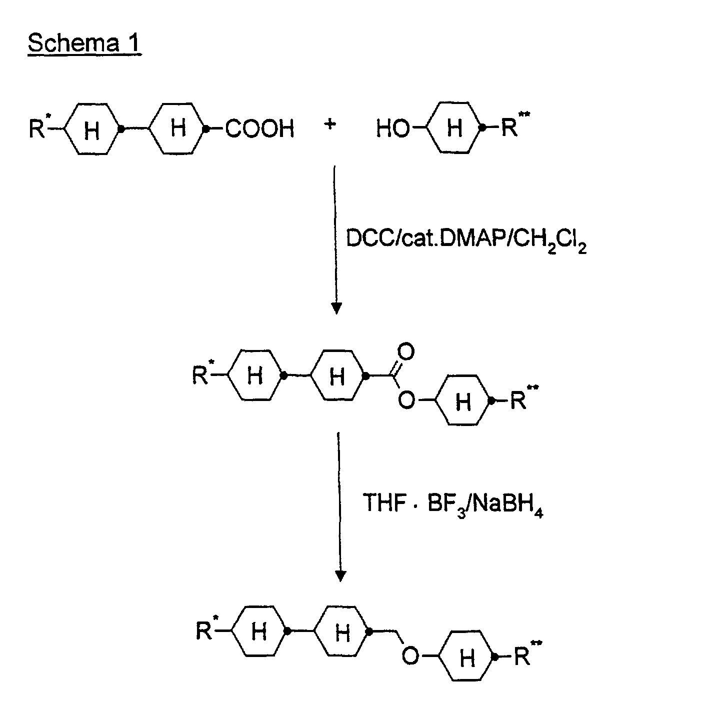

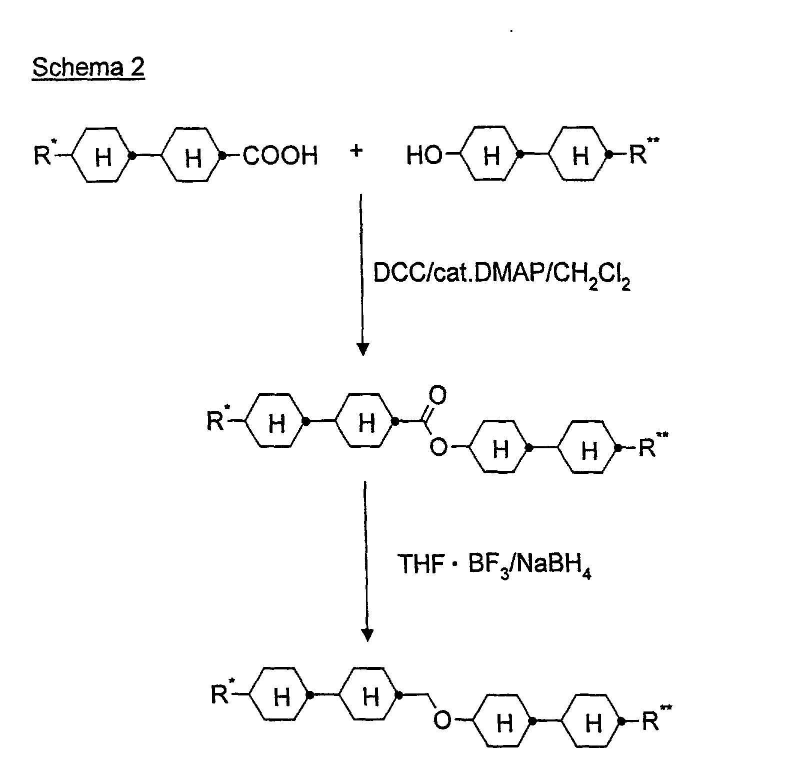

Gegenstand der Erfindung sind auch elektrooptische Anzeigen (insbesondere STN- oder MFK-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Die erfindungsgemäßen Flüssigkristallmischungen ermöglichen eine bedeutende Erweiterung des zur Verfügung stehenden Parameterraumes.

Die erzielbaren Kombinationen aus Klärpunkt, Viskosität bei tiefer Temperatur, thermischer und UV-Stabilität und dielektrischer Anisotropie übertreffen bei weitem bisherige Materialien aus dem Stand der Technik.

Die erfindungsgemäßen Flüssigkristallmischungen ermöglichen es bei Beibehaltung der nematischen Phase bis -20 °C und bevorzugt bis -30 °C, besonders bevorzugt bis -40 °C, Klärpunkte oberhalb 80 °C, vorzugsweise oberhalb 90 °C, besonders bevorzugt oberhalb 100 °C, gleichzeitig dielektrische Anisotropiewerte Δε ≥ 3, vorzugsweise ≥ 4 und einen hohen Wert für den spezifischen Widerstand zu erreichen, wodurch hervorragende STN- und MKF-Anzeigen erzielt werden können. Insbesondere sind die Mischungen durch kleine Operationsspannungen gekennzeichnet. Die TN-Schwellen liegen unterhalb 2,4 V, vorzugsweise unterhalb 2,2 V, besonders bevorzugt < 2,0 V.

Es versteht sich, daß durch geeignete Wahl der Komponenten der erfindungsgemäßen Mischungen auch höhere Klärpunkte (z.B. oberhalb 110 °C) bei höheren Schwellenspannung oder niedrigere Klärpunkte bei niedrigeren Schwellenspannungen unter Erhalt der anderen vorteilhaften Eigenschaften realisiert werden können. Ebenso können bei entsprechend wenig erhöhten Viskositäten Mischungen mit größerem Δε und somit geringeren Schwellen erhalten werden. Die erfindungsgemäßen MFK-Anzeigen arbeiten vorzugsweise im ersten Transmissionsminimum nach Gooch und Tarry [C.H. Gooch und H.A. Tarry, Electron. Lett. 10, 2-4, 1974; C.H. Gooch und H.A. Tarry, Appl. Phys., Vol. 8, 1575-1584, 1975], wobei hier neben besonders günstigen elektrooptischen Eigenschaften wie z.B. hohe Steilheit der Kennlinie und geringe Winkelabhängigkeit des Kontrastes (DE-PS 30 22 818) bei gleicher Schwellenspannung wie in einer analogen Anzeige im zweiten Minimum eine kleinerere dielektrische Anisotropie ausreichend ist. Hierdurch lassen sich unter Verwendung der erfindungsgemäßen Mischungen im ersten Minimum deutlich höhere spezifische Widerstände verwirklichen als bei Mischungen mit Cyanverbindungen. Der Fachmann kann durch geeignete Wahl der einzelnen Komponenten und deren Gewichtsanteilen mit einfachen Routinemethoden die für eine vorgegebene Schichtdicke der MFK-Anzeige erforderliche Doppelbrechung einstellen.

Die Fließviskosität ν₂₀ bei 20 °C ist vorzugsweise < 60 mm² · s⁻¹, besonders bevorzugt < 50 mm² · s⁻¹. Der nematische Phasenbereich ist vorzugsweise mindestens 90°, insbesondere mindestens 100°. Vorzugsweise erstreckt sich dieser Bereich mindestens von -20° bis +80°.

Messungen des "Capacity Holding-ratio" (HR) [S. Matsumoto et al., Liquid Crystals 5, 1320 (1989); K. Niwa et al., Proc. SID Conference, San Francisco, June 1984, p. 304 (1984); G. Weber et al., Liquid Crystals 5, 1381 (1989)] haben ergeben, daß erfindungsgemäße Mischungen enthaltend Verbindungen der Formeln IA und IB eine deutlich kleinere Abnahme des HR mit steigender Temperatur aufweisen als analoge Mischungen enthaltend anstelle den Verbindungen der Formel IB

Cyanophenylcyclohexane der Formel 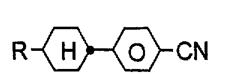 oder Ester der Formel

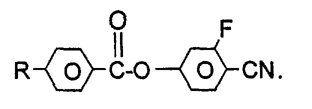

Auch die UV-Stabilität der erfindungsgemäßen Mischungen ist erheblich besser, d. h. sie zeigen eine deutlich kleinere Abnahme des HR unter UV-Belastung.

Vorzugsweise basieren die erfindungsgemäßen Medien auf mehreren (vorzugsweise zwei, drei, vier oder mehr) Verbindungen der Formel IA und/oder IB, d.h. der Anteil dieser Verbindungen ist 5-95 %, vorzugsweise 10-60 % und besonders bevorzugt im Bereich von 20-50 %.

Die einzelnen Verbindungen der Formeln IA, IB, II bis XVI und deren Unterformeln, die in den erfindungsgemäßen Medien verwendet werden können, sind entweder bekannt, oder sie können analog zu den bekannten Verbindungen hergestellt werden.

Bevorzugte Ausführungsformen sind im folgenden angegeben:
- Verbindungen der Formel IA werden vorzugsweise aus der Gruppe der Verbindungen IA1-IA10 ausgewählt: 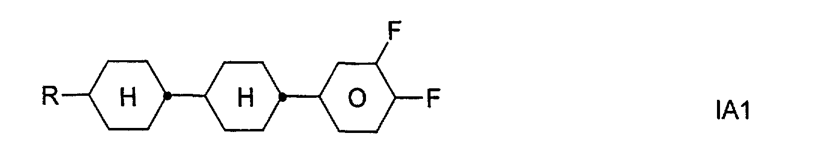 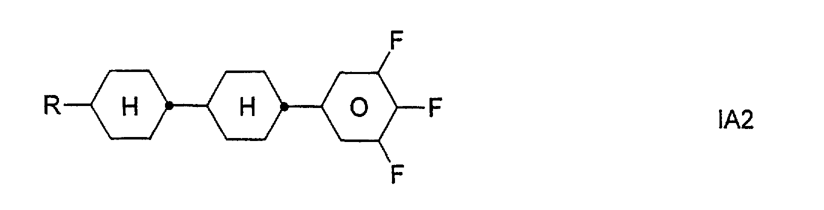 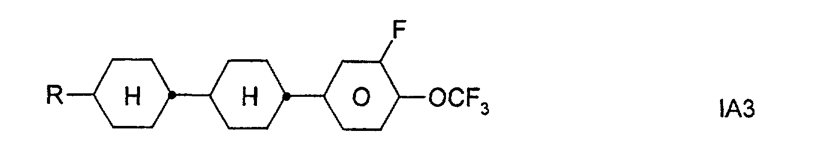 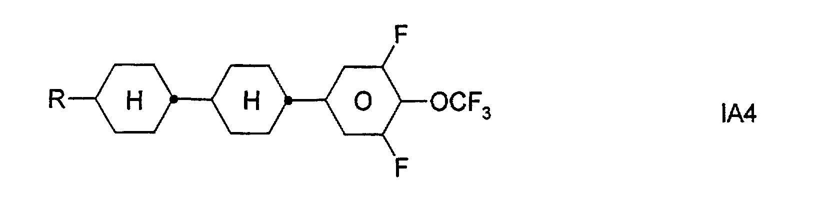 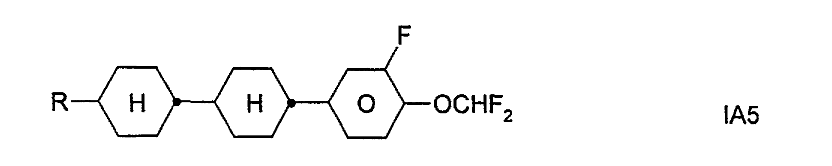 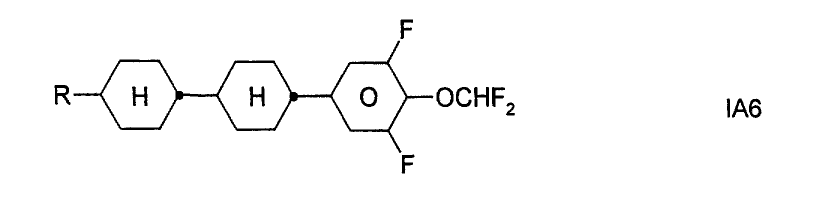 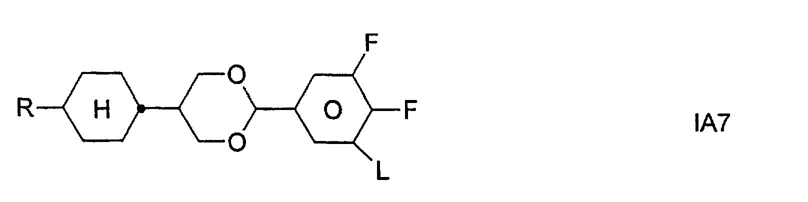 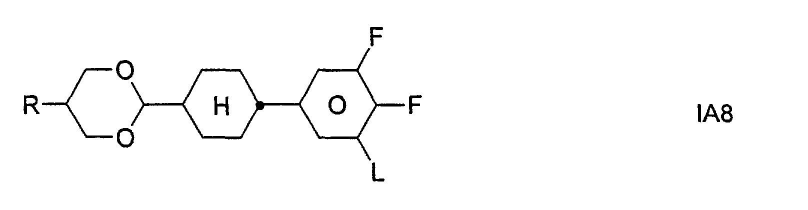 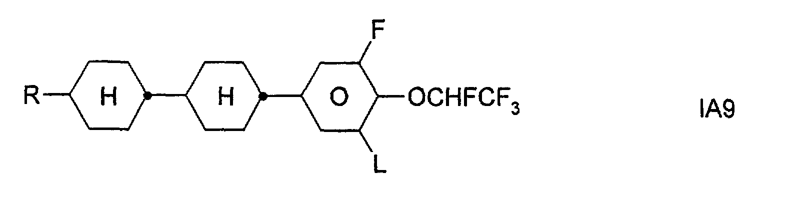 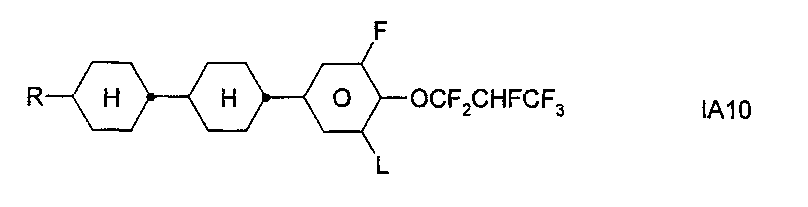 worin R die in Anspruch 1 angegebene Bedeutung besitzt, vorzugsweise jedoch ein geradkettiges Alkyl, Vinyl, 1E-Alkenyl oder 3E-Alkenyl, insbesondere Methyl, Ethyl, n-Propyl, n-Butyl, n-Pentyl oder n-Hexyl ist.
- In der Verbindung der Formel IB bedeuten R* und R** jeweils unabhängig voneinander vorzugsweise geradkettiges Alkyl, Alkoxy, Alkenyl oder Alkenyloxy, insbesondere Alkyl mit 1 bis 5 C-Atomen.
- Bevorzugte flüssigkristalline Mischungen enthalten zwei oder drei Verbindungen der Formel IB. Insbesondere bevorzugt sind Mischungen die zwei oder drei Verbindungen der Formel IB1 bis IB6 enthalten: 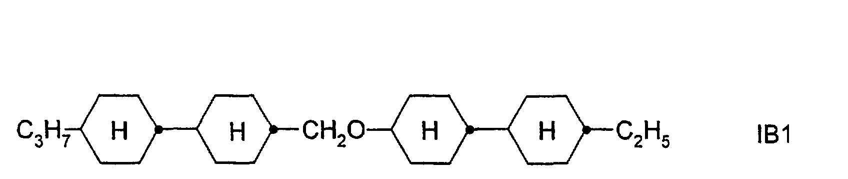 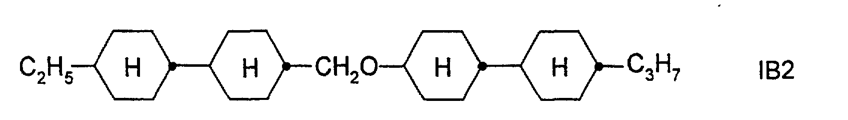 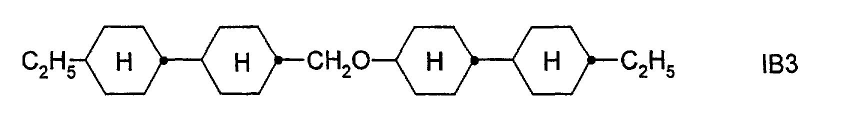 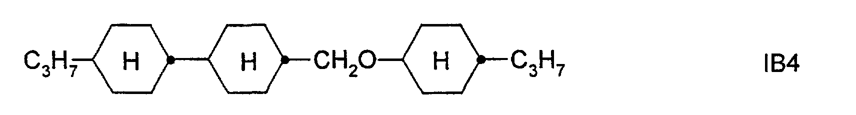 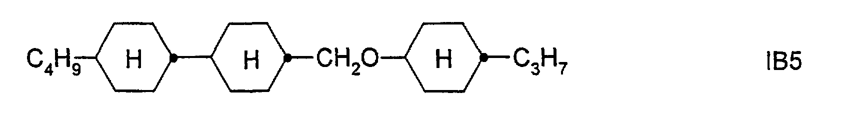 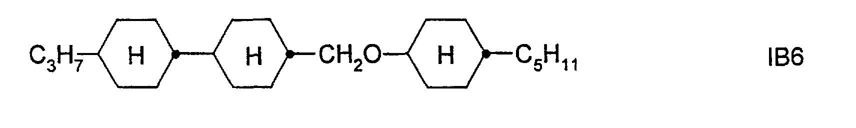
- Das Medium enthält zusätzlich eine oder mehrere Verbindungen ausgewählt aus der Gruppe bestehend aus den allgemeinen Formeln II bis VIII: 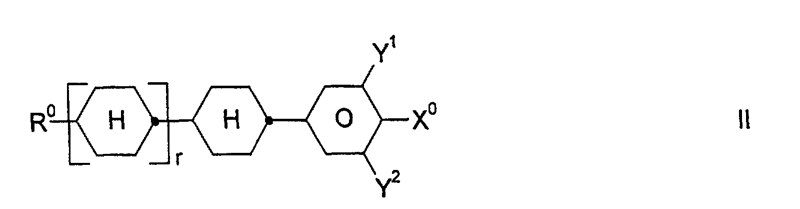 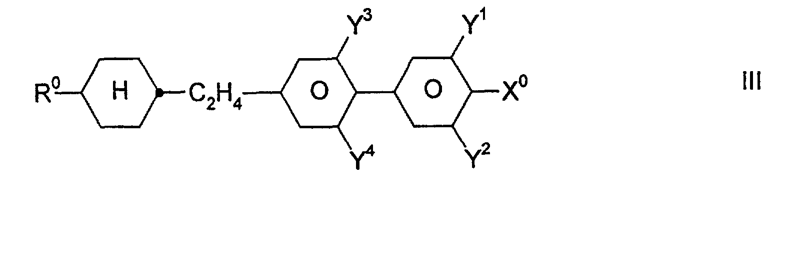 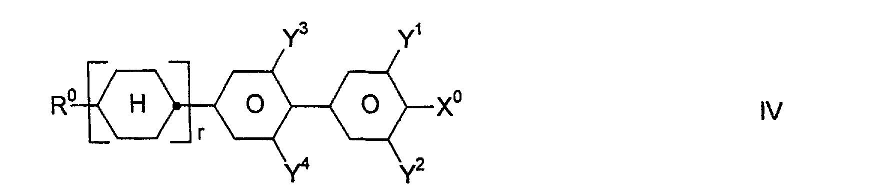 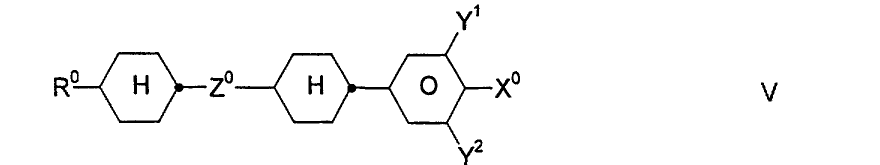 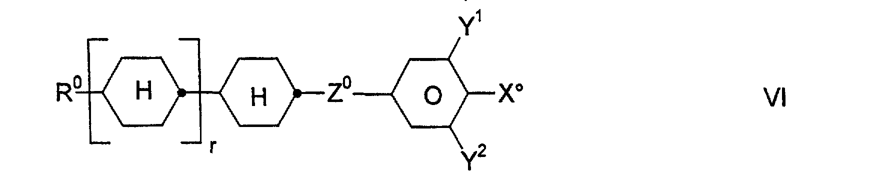 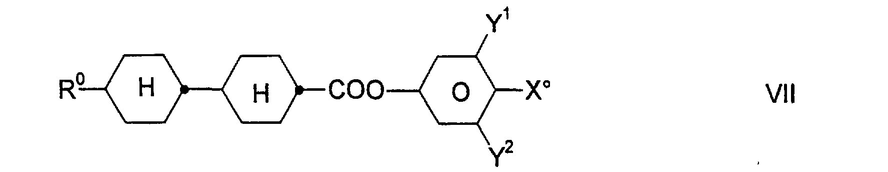 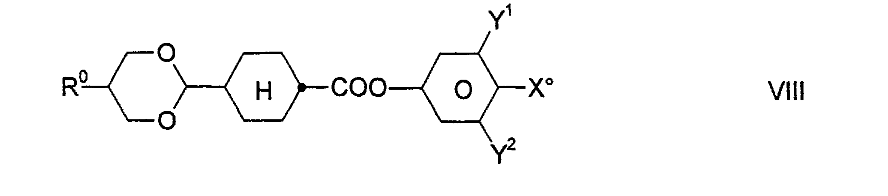
worin die einzelnen Reste die folgenden Bedeutungen haben:
- R⁰:: n-Alkyl, Oxaalkyl, Fluoralkyl, Alkenyloxy oder Alkenyl mit jeweils bis zu 9 C-Atomen
- X⁰:: F, Cl, halogeniertes Alkyl, Alkenyl oder Alkoxy mit 1 bis 6 C-Atomen,
- Z⁰:: -C₂H₄-, -CH₂O-, -OCH₂-, -CF₂O-, -OCF₂-, -CF=CF- oder -C₂F₄-
- Y¹ bis Y⁴:: jeweils unabhängig voneinander H oder F,
- r:: 0 oder 1,

Die Verbindung der Formel IV ist vorzugsweise 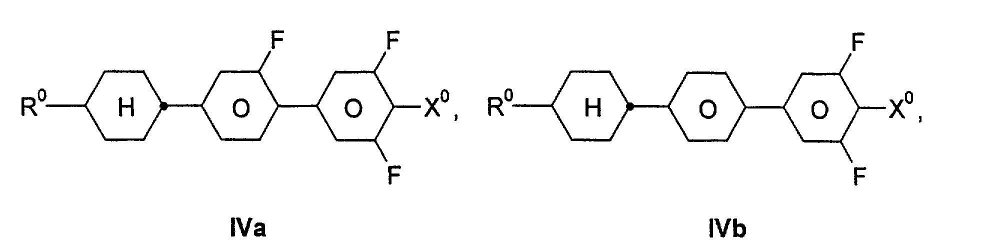 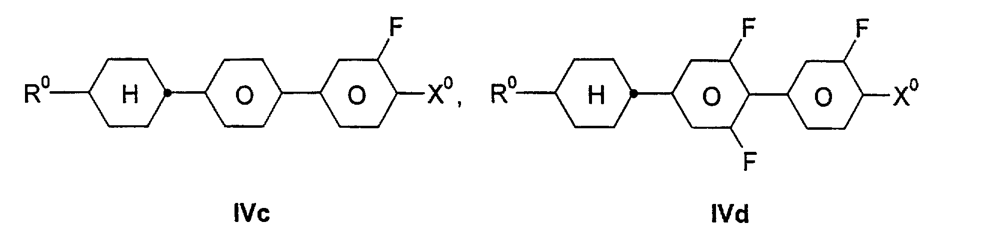 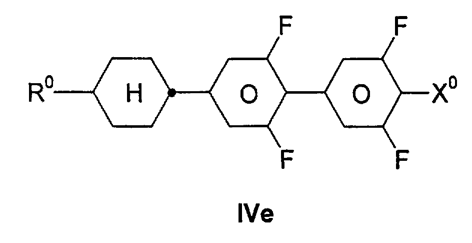 oder

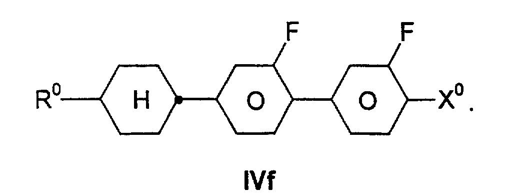

Die Verbindungen der Formeln II und IV sind dabei nicht identisch mit den Verbindungen der Formel IA.
- Das Medium enthält zusätzlich eine oder mehrere Verbindungen ausgewählt aus der Gruppe bestehend aus den allgemeinen Formeln IX bis XVI: 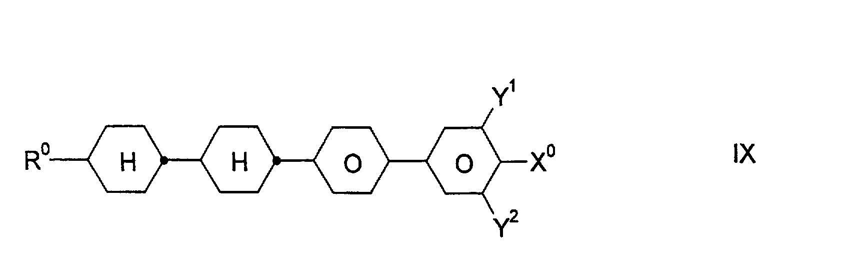 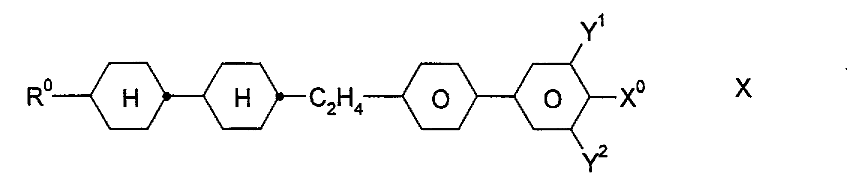 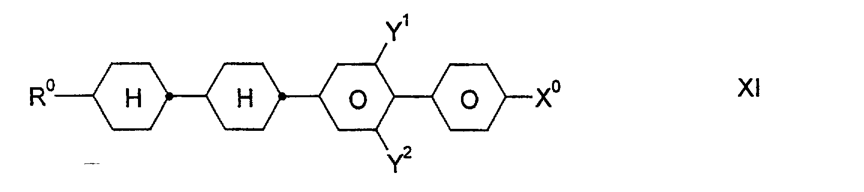 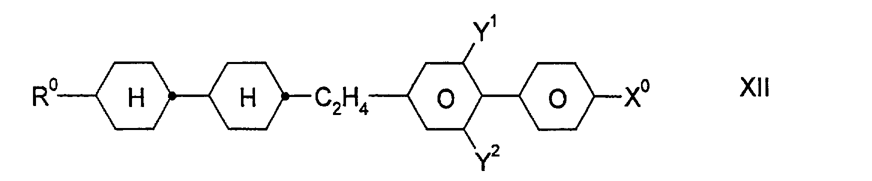 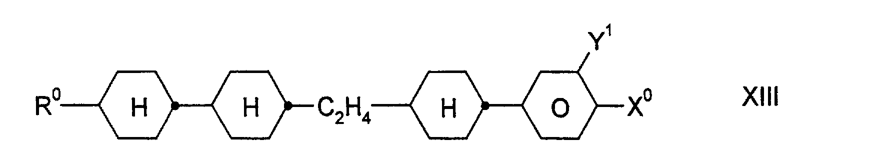 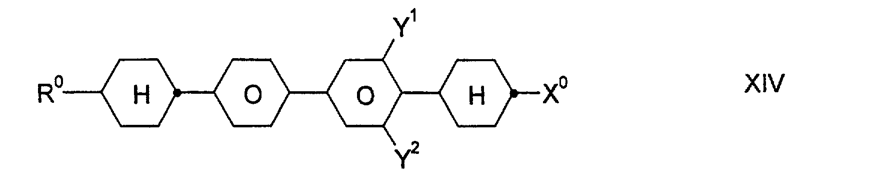 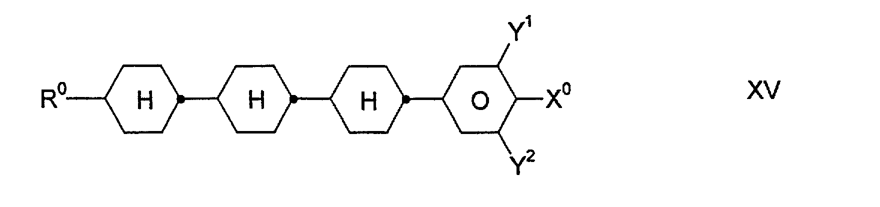 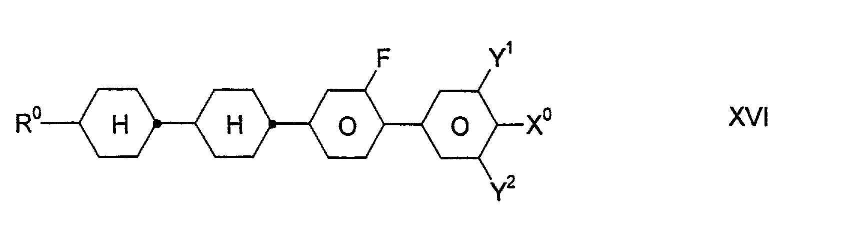 worin R⁰, X⁰, Y¹ und Y² jeweils unabhängig voneinander eine der in Anspruch 2 angegebenen Bedeutung haben. X⁰ ist vorzugsweise F, Cl, CF₃, OCF₃, OCHF₂, OCHFCF₃, OC₂F₅, ferner OCH=CF₃, OCF=CF₂, OCH=CHF, OCH=CH-CF₃, OCF₂CHF₂, C₂F₅, C₃F₇, OCH₂CH₂CF₃, R⁰ ist vorzugsweise Alkyl, Oxaalkyl, Fluoralkyl, Alkenyloxy oder Alkenyl mit jeweils bis zu 6 C-Atomen.
- Das Medium enthält zusätzlich eine oder mehrere Verbindungen der Formeln RI, RII, RIII, RIV oder RV 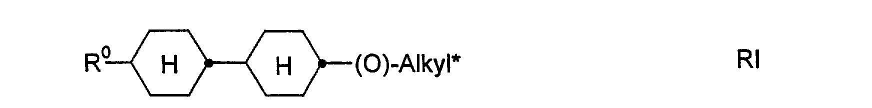 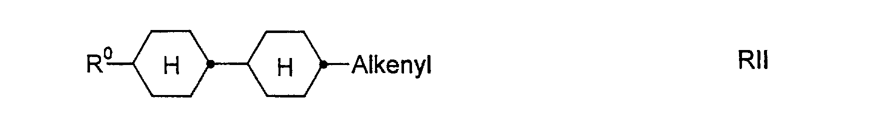 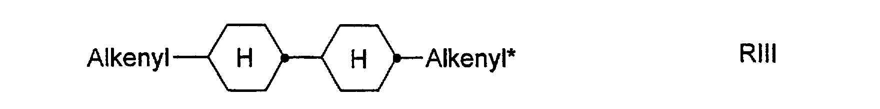 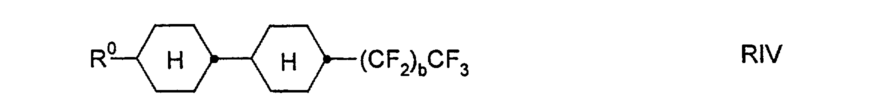 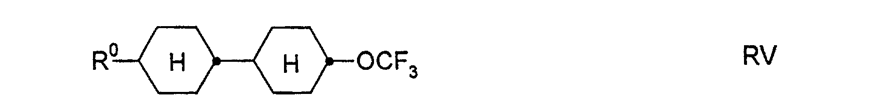 worin R⁰ die oben angegebene Bedeutung hat, vorzugsweise geradkettiges Alkyl mit 1-6 C-Atomen bedeutet und Alkenyl und Alkenyl* bedeuten geradkettiges oder verzweigte Alkenylreste mit 2-9 C-Atomen, vorzugsweise jeweils unabhängig voneinander Vinyl, 1 E-Alkenyl, 3E-Alkenyl oder 4-Alkenyl mit bis zu 9 C-Atomen; Alkyl und Alkyl* bedeuten geradkettiges Alkyl mit 1 bis 9 C-Atomen; b ist 0,1 oder 2.
- Der Anteil an Verbindungen der Formeln IA, IB und II bis VI zusammen beträgt im Gesamtgemisch mindestens 50 Gew.-%;
- Der Anteil an Verbindungen der Formeln IA und IB beträgt im Gesamtgemisch 10 bis 100 Gew.-%;
- Der Anteil der Verbindungen der Formel IB beträgt im Gesamtgemisch 2 bis 30 Gew.-%, vorzugsweise 4 bis 20 Gew.-%;
- Der Anteil an Verbindungen der Formeln II bis VIII im Gesamtgemisch beträgt 10 bis 50 Gew.-%; 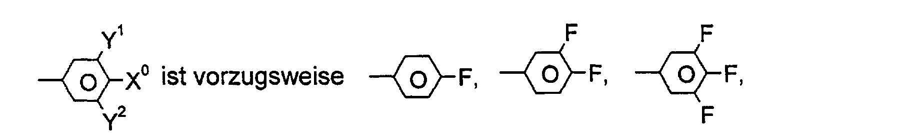 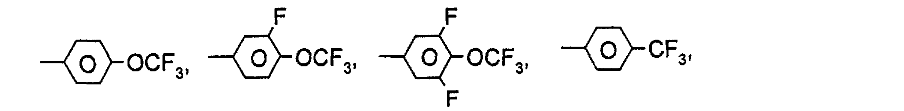 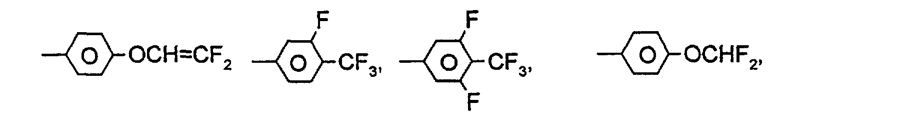 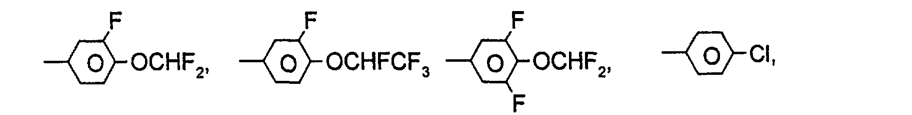 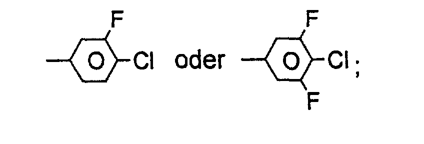
- Das Medium enthält vorzugsweise neben ein oder mehreren Verbindungen der Formeln IA und IB ein oder mehrere Verbindungen, vorzugsweise zwei, drei oder vier Verbindungen, der Formeln II, III, IV, V, VI, VII und/oder VIII;
- Das erfindungsgemäße Medium enthält vorzugsweise ein oder mehrere Verbindungen der Formeln 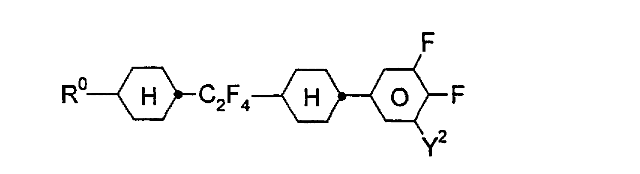 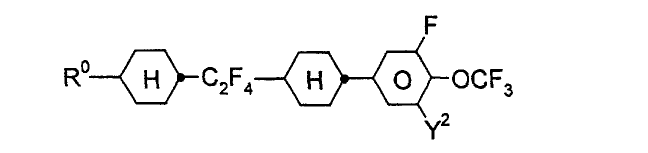 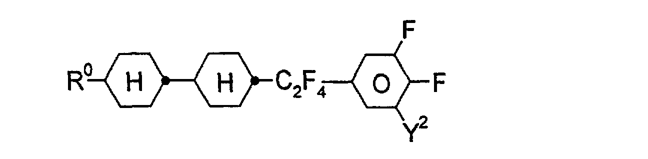 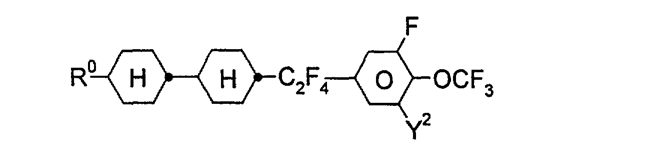 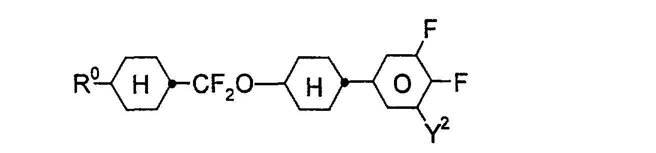 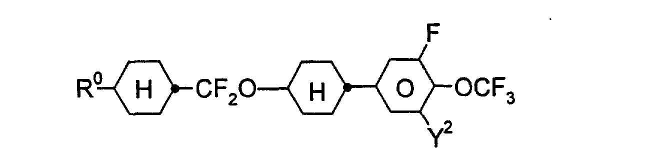 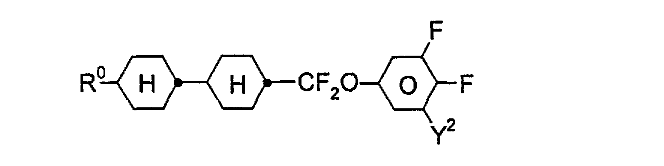 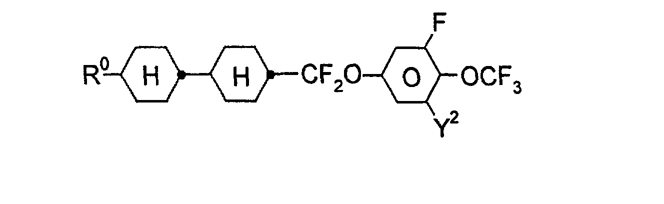 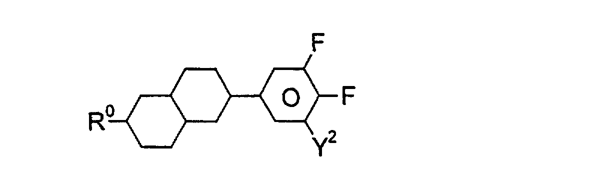 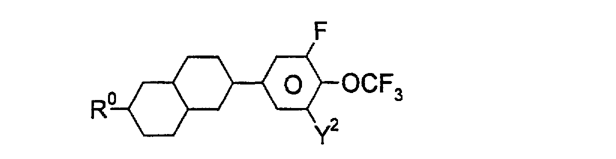 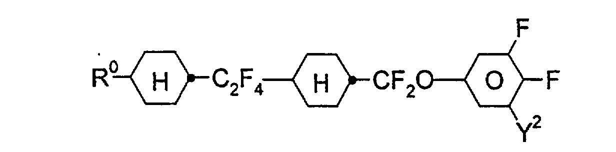 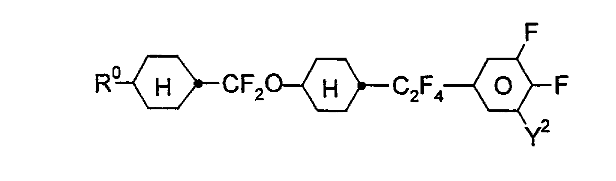
- R⁰ ist geradkettiges Alkyl oder Alkenyl mit 2 bis 7 C-Atomen;
- Das Medium besteht im wesentlichen aus Verbindungen der Formeln IA, IB und II bis VIII;
- Das Medium enthält weitere Verbindungen, vorzugsweise ausgewählt aus der folgenden Gruppe bestehend aus den allgemeinen Formeln XVII bis XX: 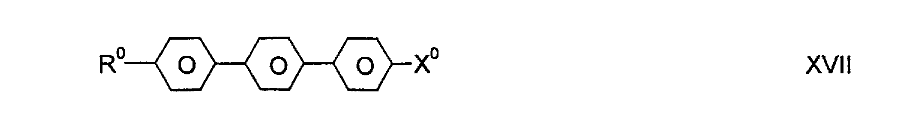 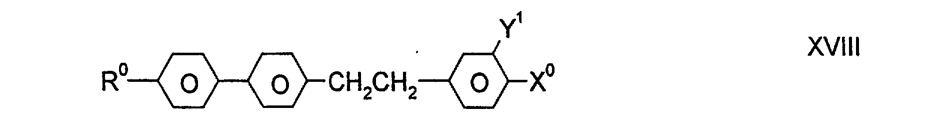 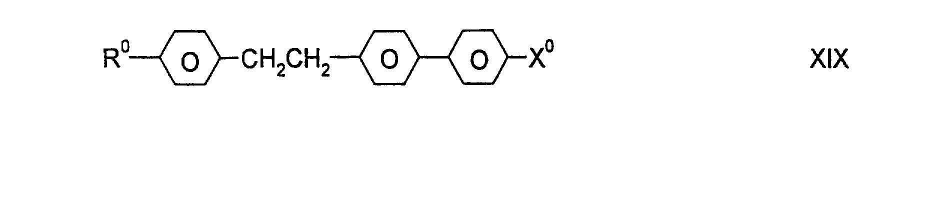 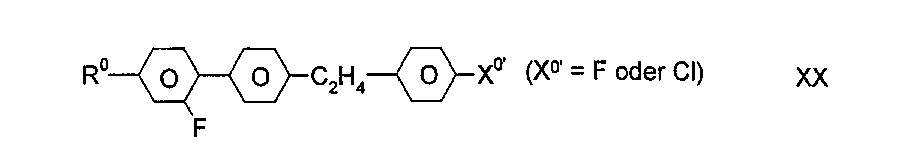 worin R⁰, Y¹ und X⁰ die oben angegebenen Bedeutungen haben und die 1,4-Phenylenringe durch ein oder zwei CN, Chlor oder Fluor substituiert sein können. Vorzugsweise sind die 1,4-Phenylenringe ein- oder mehrfach durch Fluoratome substituiert.
- Das Gewichtsverhältnis (IA + IB): (II + III + IV + V + VI + VII + VIII) ist vorzugsweise 1 : 10 bis 10 : 1.
- Das Medium besteht im wesentlichen aus Verbindungen ausgewählt aus der Gruppe bestehend aus den allgemeinen Formeln IA, IB, II bis XVI, RI, RII, RIII, RIV und/oder RV.
- Der Anteil an Verbindungen der Formeln RI, RII und/oder RIII im Gesamtgemisch liegt bei 5-60 Gew.%, vorzugsweise bei 10-40 Gew.%.

Es wurde gefunden, daß bereits ein relativ geringer Anteil an Verbindungen der Formeln IA und IB im Gemisch mit üblichen Flüssigkristallmaterialien, insbesondere jedoch mit einer oder mehreren Verbindungen der Formel II, III, IV, V, VI, VII und/oder VIII zu einer beträchtlichen Erniedrigung der Schwellenspannung und zu niedrigen Werten für die Doppelbrechung führt, wobei gleichzeitig breite nematische Phasen mit tiefen Übergangstemperaturen smektisch-nematisch beobachtet werden, wodurch die Lagerstabilität verbessert wird. Bevorzugt sind insbesondere Mischungen, die neben ein oder mehrerer Verbindungen der Formel IA und IB ein oder mehrere Verbindungen der Formel IV enthalten, insbesondere Verbindungen der Formel IVa, worin X⁰ F oder OCF₃ bedeutet. Die Verbindungen der Formeln IA, IB, II bis VIII sind farblos, stabil und untereinander und mit anderen Flüssigkristallmaterialien gut mischbar.

Der Ausdruck "Alkyl" oder "Alkyl*" umfaßt geradkettige und verzweigte Alkylgruppen mit 1-6 Kohlenstoffatomen, insbesondere die geradkettigen Gruppen Methyl, Ethyl, Propyl, Butyl, Pentyl und Hexyl. Gruppen mit 2-5 Kohlenstoffatomen sind im allgemeinen bevorzugt.

Der Ausdruck "Alkenyl" bzw. "Alkenyl*" umfaßt geradkettige und verzweigte Alkenylgruppen mit 2-9 Kohlenstoffatomen, insbesondere die geradkettigen Gruppen. Besonders Alkenylgruppen sind C₂-C₇-1E-Alkenyl, C₄-C₇-3E-Alkenyl, C₅-C₇-4-Alkenyl, C₆-C₇-5-Alkenyl und C₇-6-Alkenyl, insbesondere C₂-C₇-1E-Alkenyl, C₄-C₇-3E-Alkenyl und C₅-C₇-4-Alkenyl. Beispiele bevorzugter Alkenylgruppen sind Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfaßt vorzugsweise geradkettige Gruppen mit endständigen Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl" umfaßt vorzugsweise geradkettige Reste der Formel CₙH₂ₙ₊₁-O-(CH₂)ₘ, worin n und m jeweils unabhängig voneinander 1 bis 6 bedeuten. Vorzugsweise ist n = 1 und m 1 bis 6.Durch geeignete Wahl der Bedeutungen von R⁰ und X⁰ können die Ansprechzeiten, die Schwellenspannung, die Steilheit der Transmissionskennlinien etc. in gewünschter Weise modifiziert werden. Beispielsweise führen 1E-Alkenylreste, 3E-Alkenylreste, 2E-Alkenyloxyreste und dergleichen in der Regel zu kürzeren Ansprechzeiten, verbesserten nematischen Tendenzen und einem höheren Verhältnis der elastischen Konstanten k₃₃ (bend) und k₁₁ (splay) im Vergleich zu Alkyl- bzw. Alkoxyresten. 4-Alkenylreste, 3-Alkenylreste und dergleichen ergeben im allgemeinen tiefere Schwellenspannungen und kleinere Werte von k₃₃/k₁₁ im Vergleich zu Alkyl- und Alkoxyresten.

Eine -CH₂CH₂-Gruppe führt im allgemeinen zu höheren Werten von k₃₃/k₁₁ im Vergleich zu einer einfachen Kovalenzbindung. Höhere Werte von k₃₃/k₁₁ ermöglichen z.B. flachere Transmissionskennlinien in TN-Zellen mit 90° Verdrillung (zur Erzielung von Grautönen) und steilere Transmissionskennlinien in STN-, SBE- und OMI-Zellen (höhere Multiplexierbarkeit) und umgekehrt.

Das optimale Mengenverhältnis der Verbindungen der Formeln (IA + IB) und II + III + IV + V + VI + VII + VIII hängt weitgehend von den gewünschten Eigenschaften von der Wahl der Komponenten der Formeln IA, IB, II, III, IV, V, VI, VII und/oder VIII und von der Wahl weiterer gegebenenfalls vorhandener Komponenten ab. Geeignete Mengenverhältnisse innerhalb des oben angegebenen Bereichs können von Fall zu Fall leicht ermittelt werden.

Die Gesamtmenge an Verbindungen der Formeln IA, IB, II bis XVI in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die Ansprechzeiten und die Schwellenspannung ist jedoch in der Regel umso größer je höher die Gesamtkonzentration an Verbindungen der Formeln IA, IB, II bis XVI ist.

In einer besonders bevorzugten Ausführungsform enthalten die erfindungsgemäßen Medien Verbindungen der Formel II bis VIII (vorzugsweise II, III und/oder IV, insbesondere IVa), worin X⁰ F, OCF₃, OCHF₂, OCH=CF₂, OCF=CF₂ oder OCF₂-CF₂H bedeutet. Eine günstige synergistische Wirkung mit den Verbindungen der Formel IA und IB führt zu besonders vorteilhaften Eigenschaften.

Der Aufbau der erfindungsgemäßen MFK-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefaßt und umfaßt auch alle Abwandlungen und Modifikationen der MFK-Anzeige, insbesondere auch Matrix-Anzeigeelemente auf Basis poly-Si TFT oder MIM.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die Herstellung der erfindungsgemäß verwendbaren Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation.

Die Dielektrika können auch weitere, dem Fachmann bekannte und in der Literatur beschriebene Zusätze enthalten. Beispielsweise können 0-15 % pleochroitische Farbstoffe oder chirale Dotierstoffe zugesetzt werden.

C bedeutet eine kristalline, S eine smektische, S_{C} eine smektisch C, N eine nematische und 1 die isotrope Phase.

V₁₀ bezeichnet die Spannung für 10 % Transmission (Blickrichtung senkrecht zur Plattenoberfläche). tₒₙ bezeichnet die Einschaltzeit und t_{off} die Ausschaltzeit bei einer Betriebsspannung entsprechend dem 2,5fachen Wert von V₁₀. Δn bezeichnet die optische Anisotropie und nₒ den Brechungsindex. Δε bezeichnet die dielektrische Anisotropie (Δε = ε_{∥} - ε_{⊥}, wobei **ε**_{**∥**} die Dielektrizitätskonstante parallel zu den Moleküllängsachsen und ε_{⊥} die Dielektrizitätskonstante senkrecht dazu bedeutet). Die elektrooptischen Daten wurden in einer TN-Zelle im 1. Minimum (d.h. bei einem d Δn-Wert von 0,5) bei 20 °C gemessen, sofern nicht ausdrücklich etwas anderes angegeben wird. Die optischen Daten wurden bei 20 °C gemessen, sofern nicht ausdrücklich etwas anderes angegeben wird.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen; n und m bedeuten unabhängig voneinander ganze Zahlen, vorzugsweise 0, 1, 2, 3, 4, 5, 6, 7, 8, 9, 10, 11 oder 12. Die Codierung gemäß Tabelle B versteht sich von selbst. In Tabelle A ist nur das Acronym für den Grundkörper angegeben.

Im Einzelfall folgt getrennt vom Acronym für den Grundkörper mit einem Strich ein Code für die Substituenten R¹, R², L¹ und L²:

| Code für R¹, R², L¹, L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nCl | CₙH₂ₙ₊₁ | Cl | H | H |
| nF.F | CₙH₂ₙ₊₁ | F | H | F |
| nF.F.F | CₙH₂ₙ₊₁ | F | F | F |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rVsF | CᵣH₂ᵣ₊₁-CH=CH-CₛH₂ₛ- | F | H | H |
| rEsN | CᵣH₂ᵣ₊₁-O-CₛH₂ₛ- | CN | H | H |
| nAm | CₙH₂ₙ₊₁ | COOCₘH₂ₘ₊₁ | H | H |

Bevorzugte Mischungskomponenten finden sich in den Tabellen A und B.

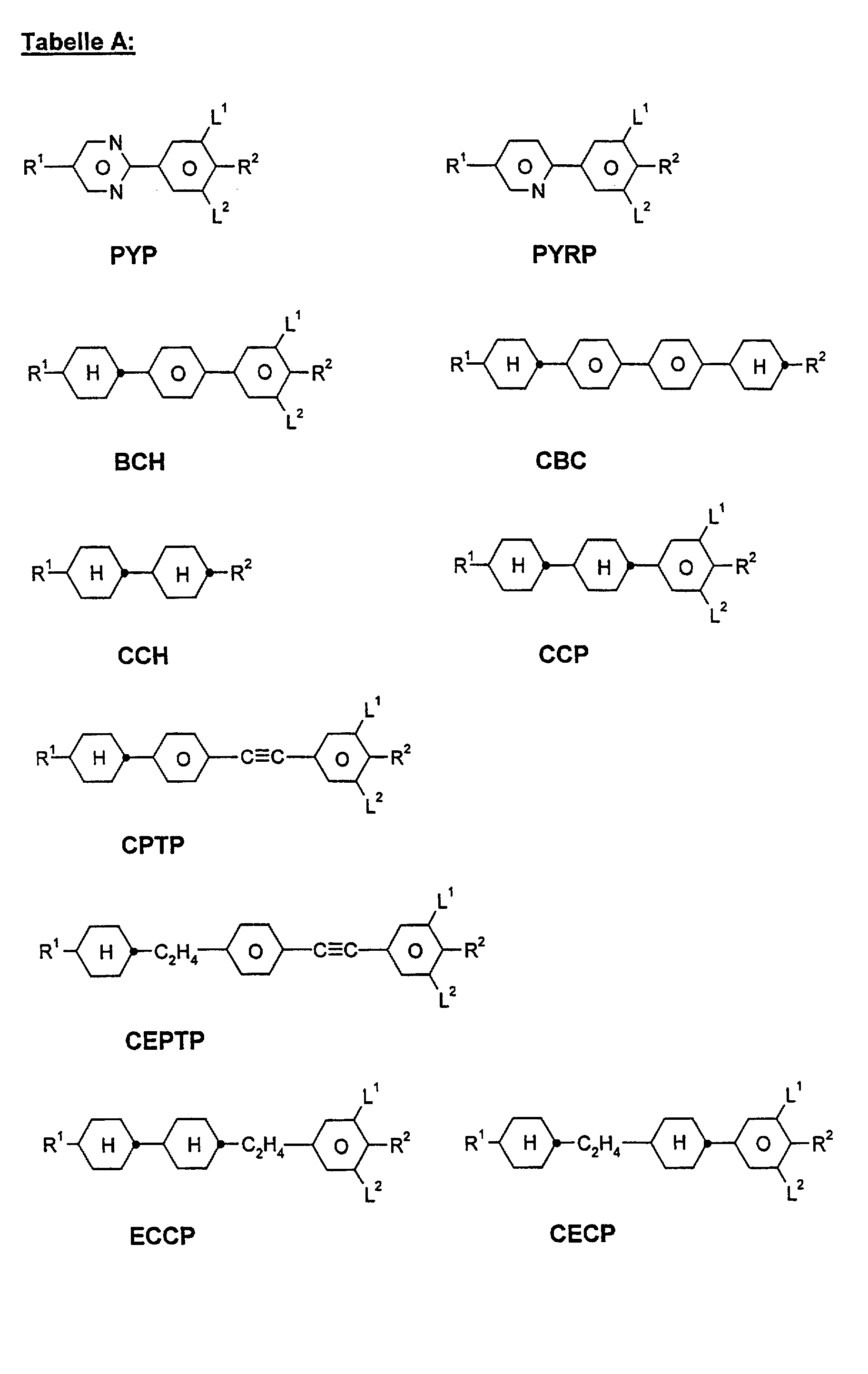

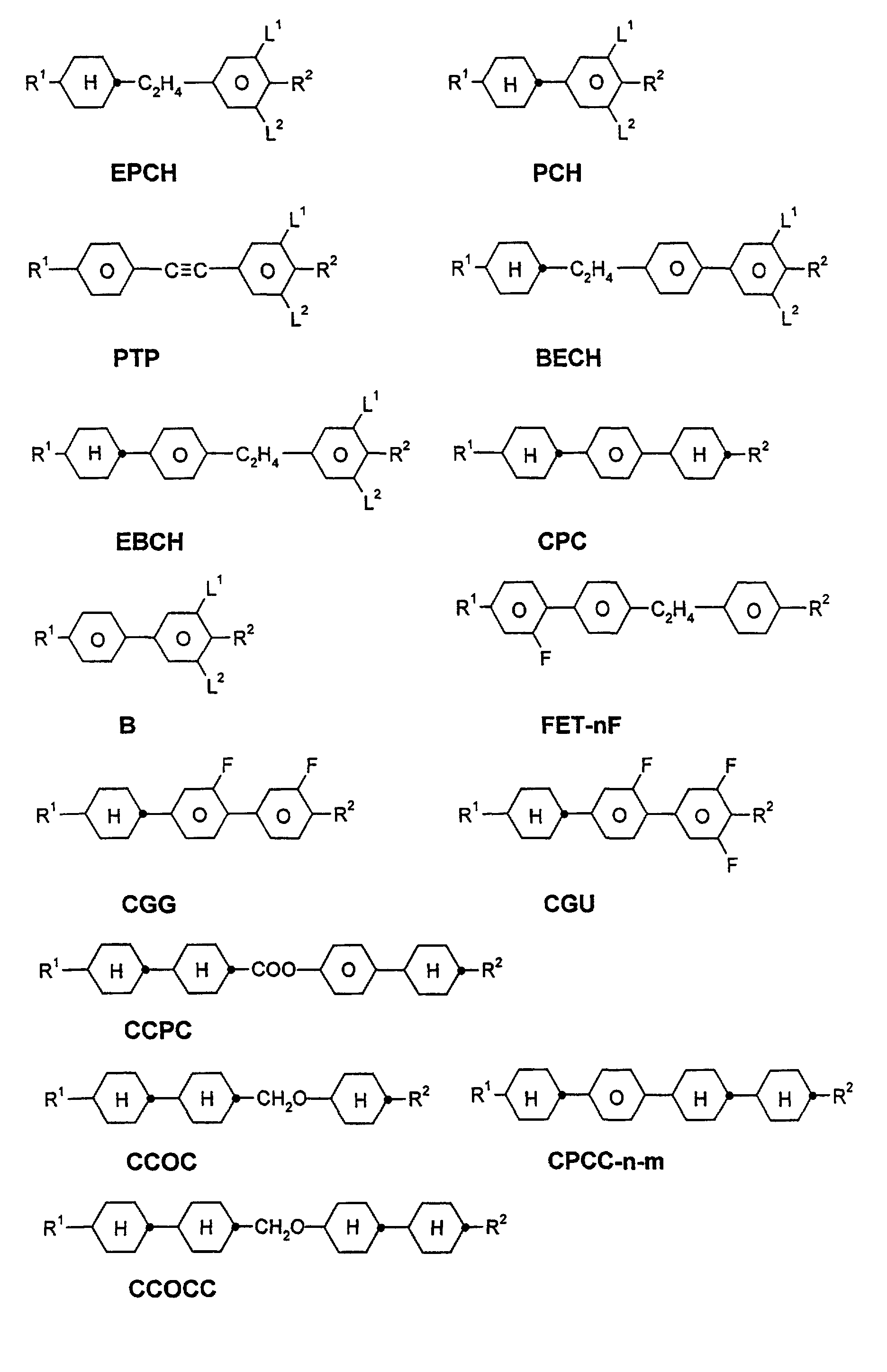

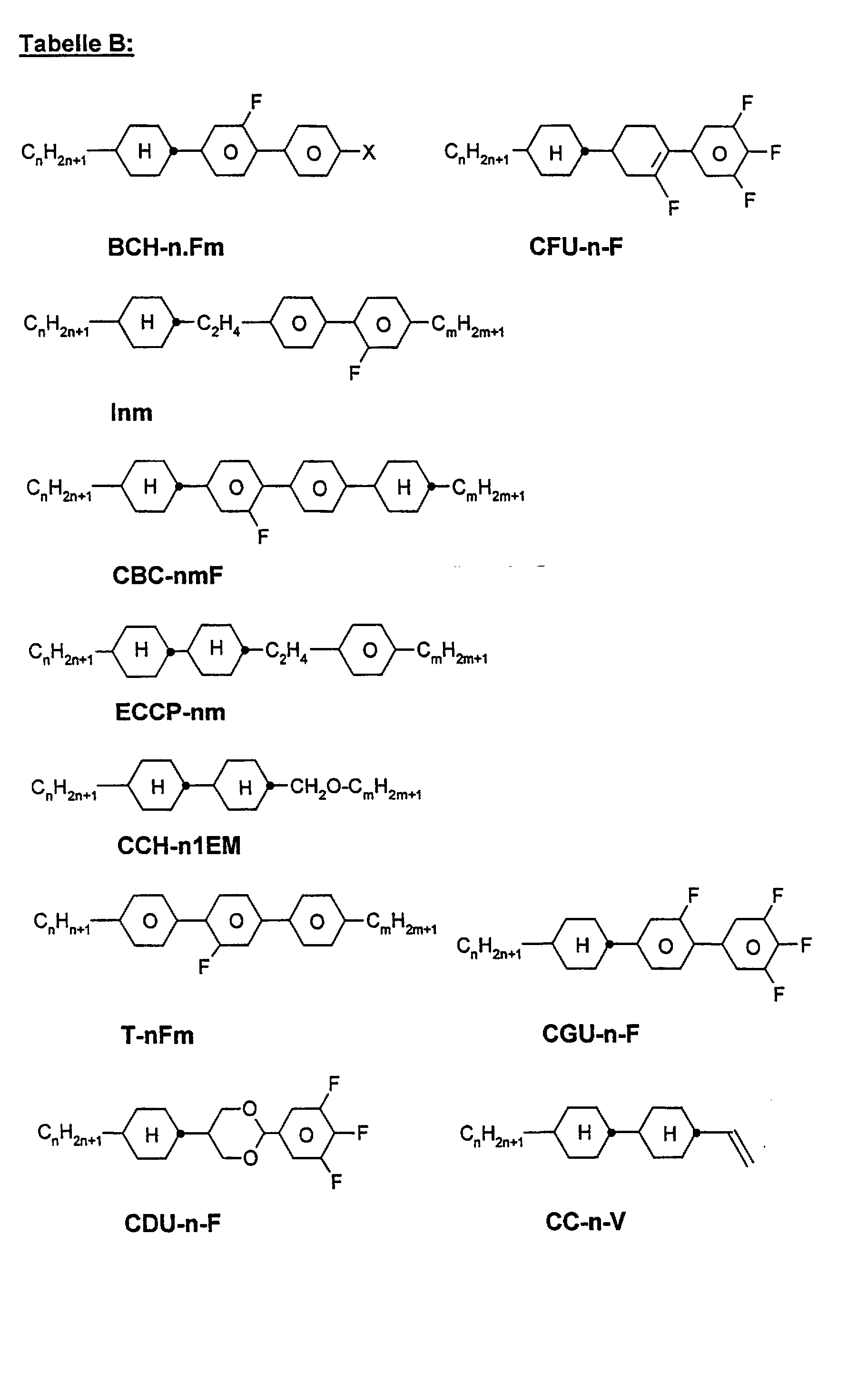

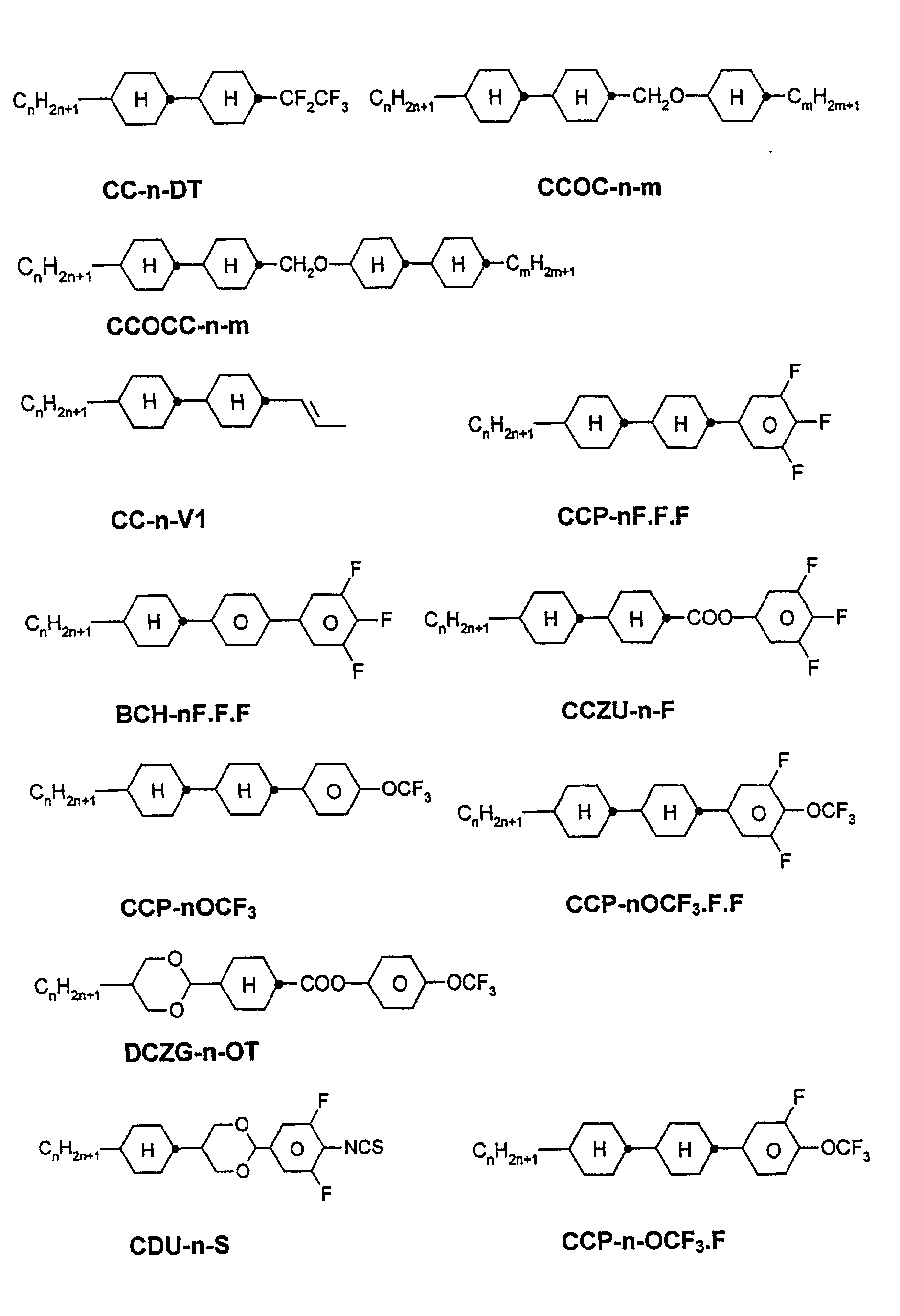

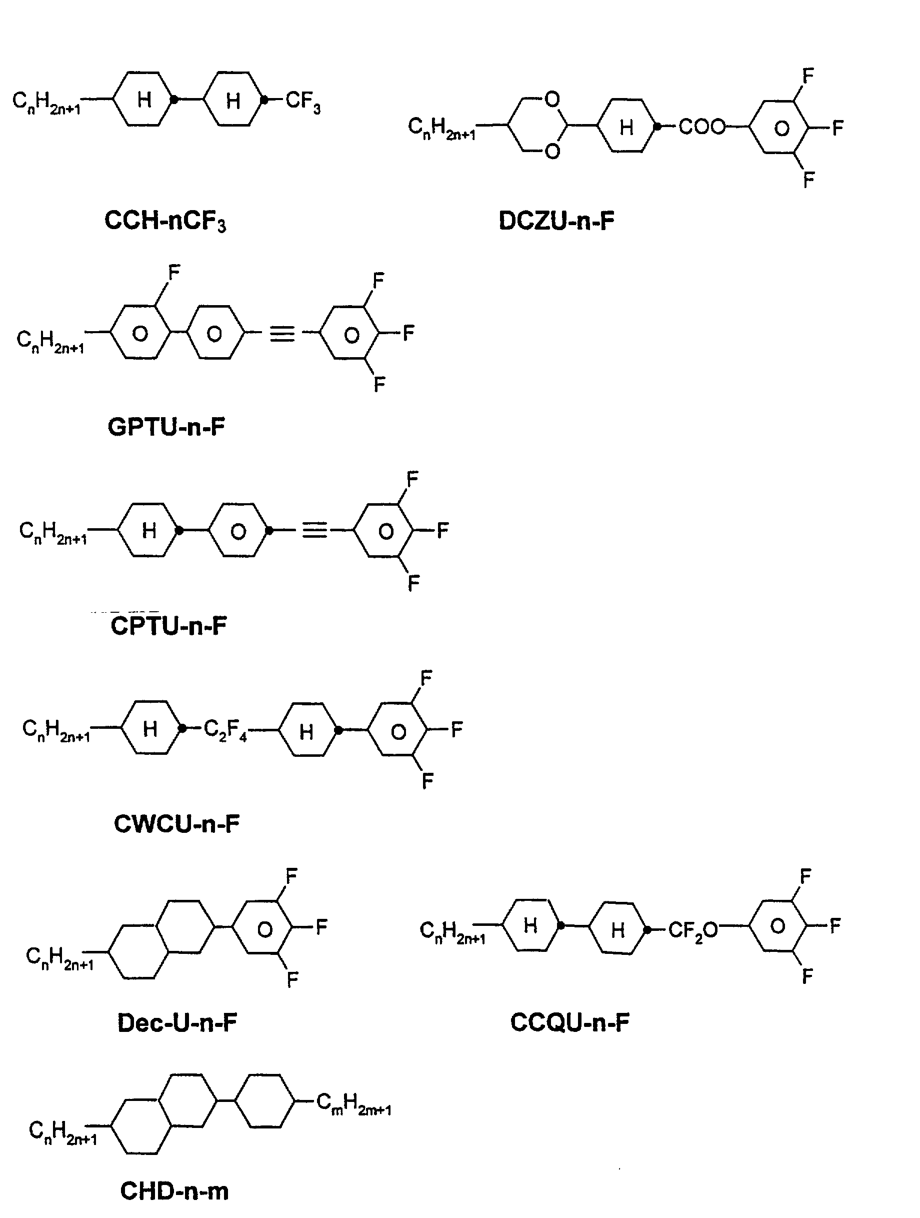

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C). Die Fließviskosität ν₂₀ (mm²/sec) und die Rotationsviskosität γ₁ [mPa · s] wurden jeweils bei 20 °C bestimmt.

"Übliche Aufarbeitung" bedeutet: man gibt gegebenenfalls Wasser hinzu, extrahiert mit Dichlormethan, Diethylether, MethylMethyl-tert.Butylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:
- n-BuLi: 1,6 molare Lösung von n-Butyllithium in n-Hexan
- DMAP: 4-(Dimethylamino)-Pyridin
- THF: Tetrahydrofuran
- DCC: N,N'-Dicyclohexylcarbodiimid

### Beispiel 1

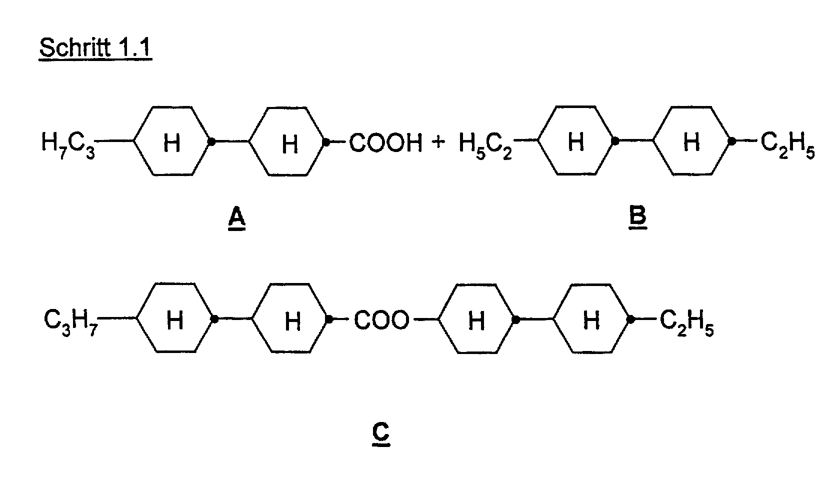

80 mmol A, 80 mmol B, 0,977 g 4-(Dimethylamino)-pyridin in 200 ml Toluol werden bei 5 °C vorgelegt. Bei 5-10 °C wird eine Lösung von 88 mmol N,N'-Dicyclohexylcarbodiimid in 70 ml Toluol zugetropft. Über Nacht läßt man bei Raumtemperatur rühren.

Zuletzt wird wie üblich angearbeitet. Das Produkt wird aus Essigester umkristallisiert. 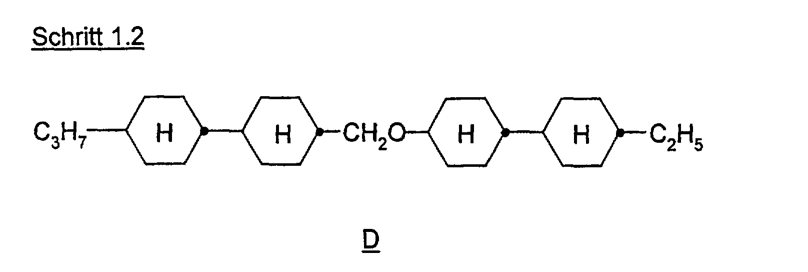 45 mmol C werden in 200 ml abs. THF gelöst und unter Eiskühlung werden bei 15-25 °C 157,5 mmol Bortrifluorid-THF-Komplex zugetropft. Zu dieser Lösung wird anschließend eine Lösung von 112,5 mmol Natriumborhydrid in 100 ml Diethylenglycoldimethylether bei 15-25 °C zugetropft. Man läßt auf Raumtemperatur erwärmen und läßt weitere 4 h rühren. Zuletzt wird wie üblich aufgearbeitet. Das Produkt wird aus Essigester umkristallisiert.

Analog werden die folgenden Verbindungen hergestellt:

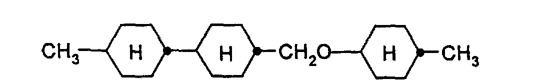

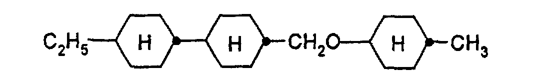

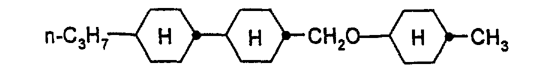

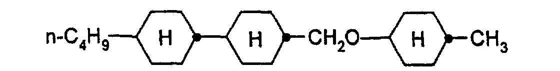

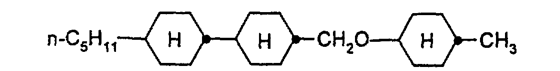

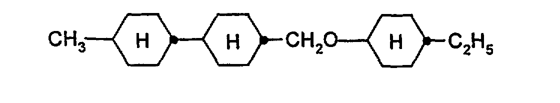

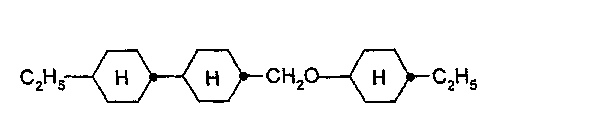

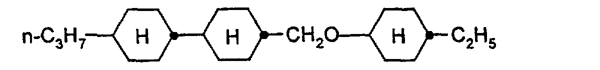

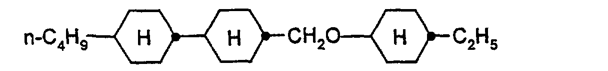

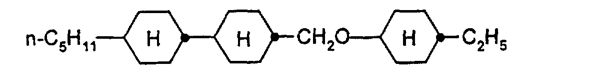

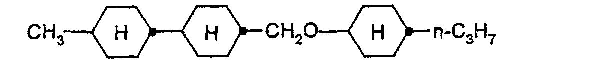

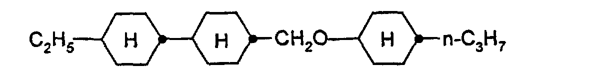

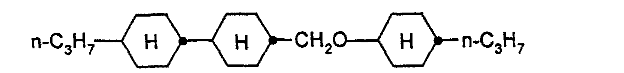

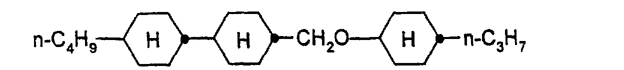

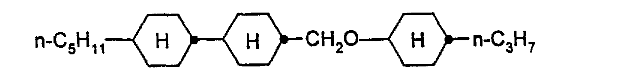

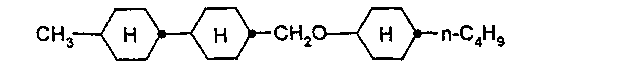

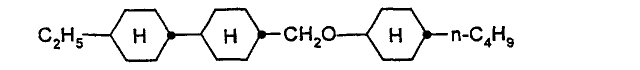

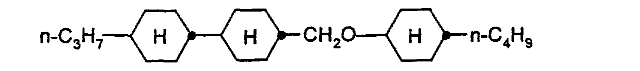

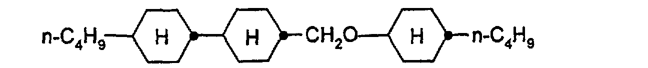

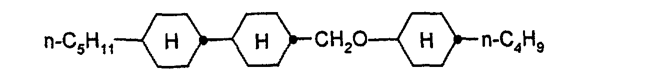

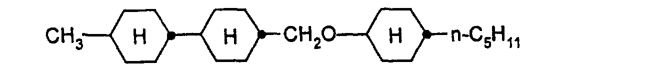

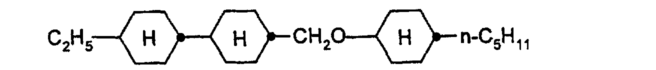

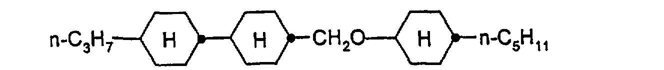

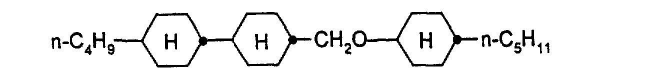

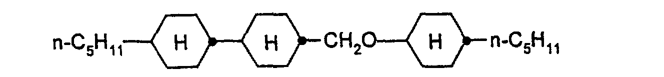

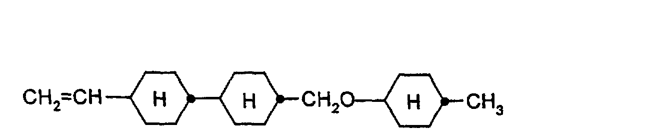

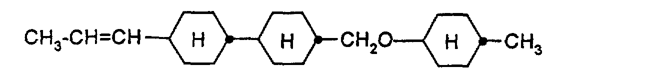

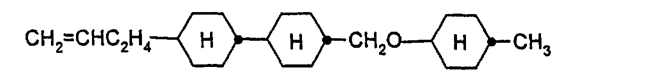

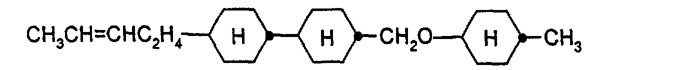

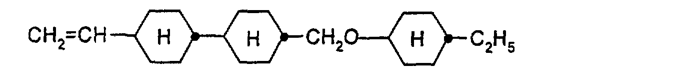

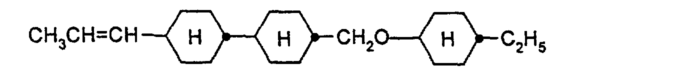

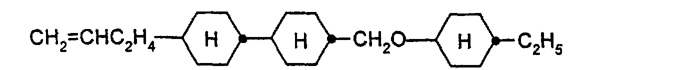

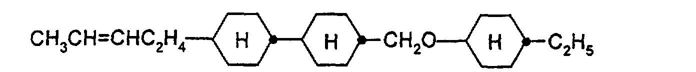

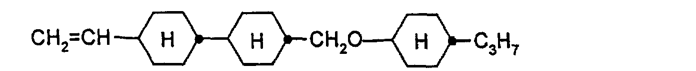

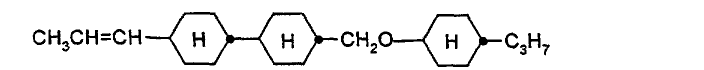

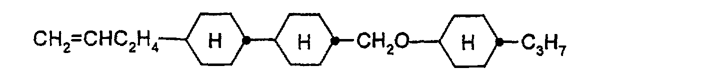

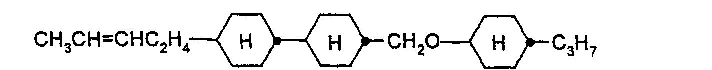

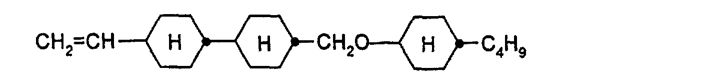

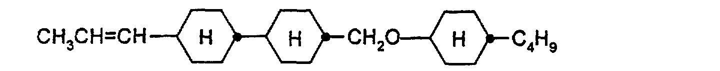

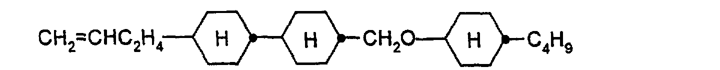

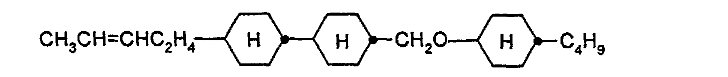

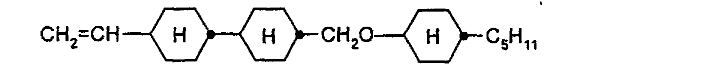

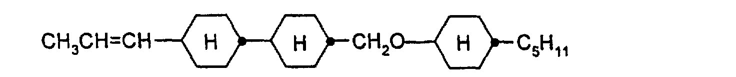

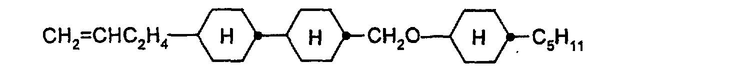

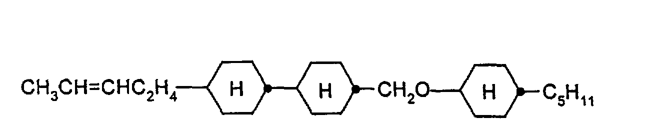

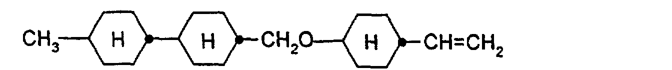

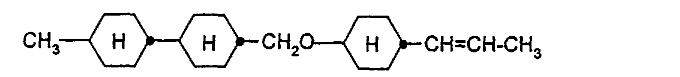

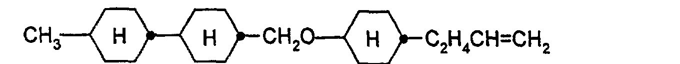

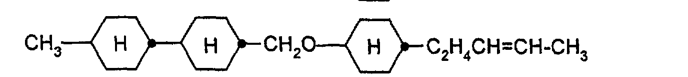

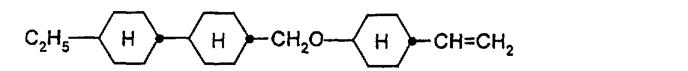

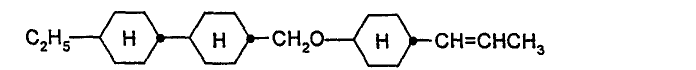

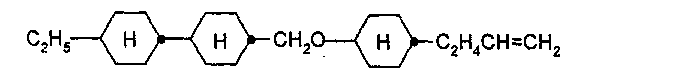

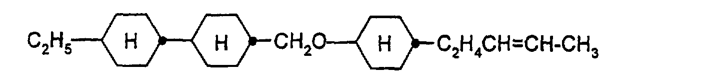

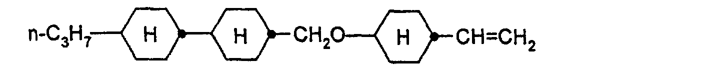

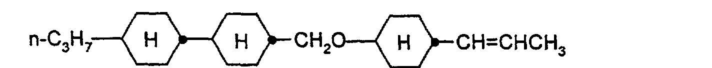

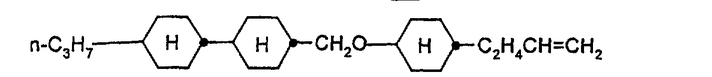

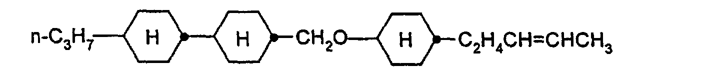

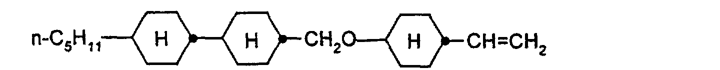

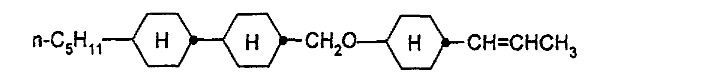

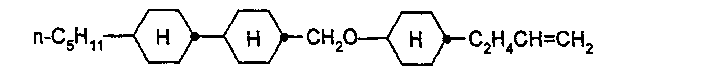

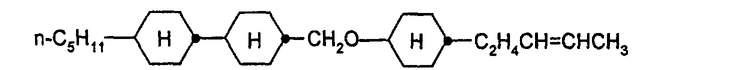

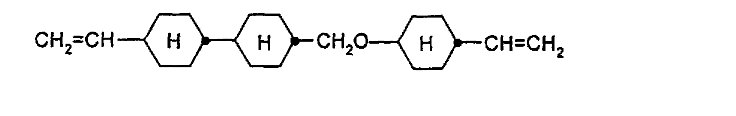

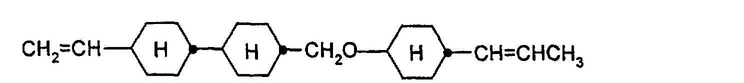

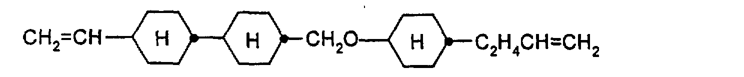

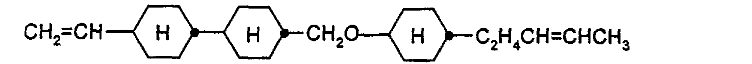

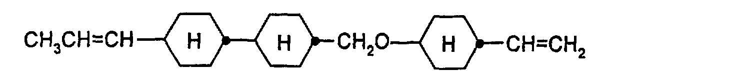

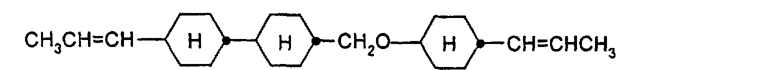

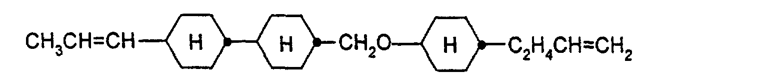

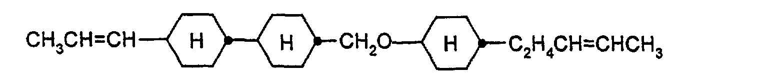

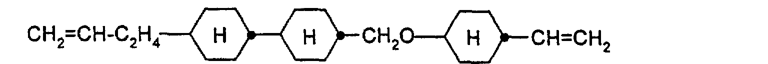

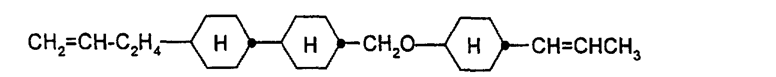

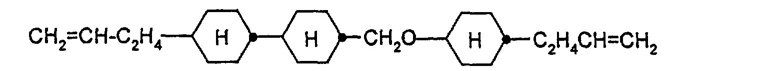

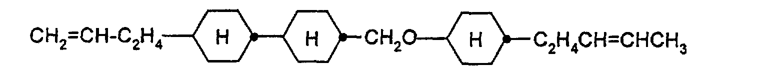

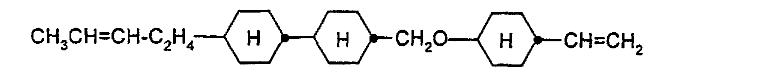

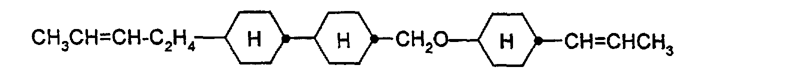

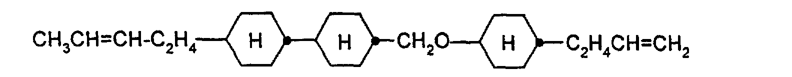

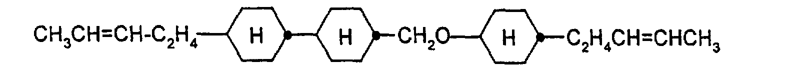

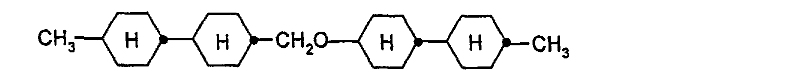

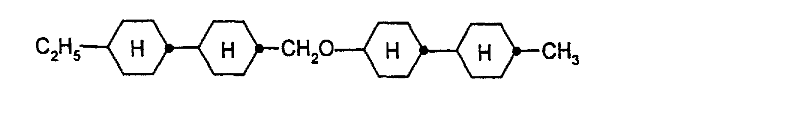

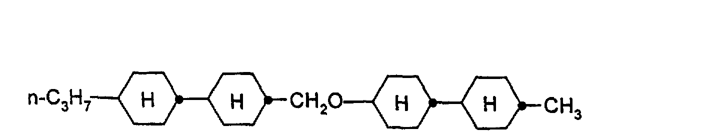

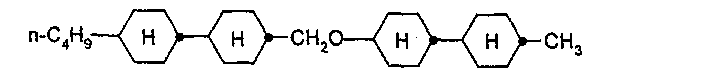

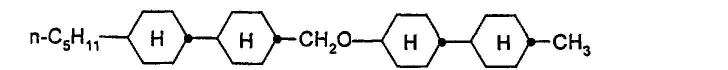

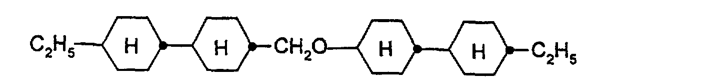

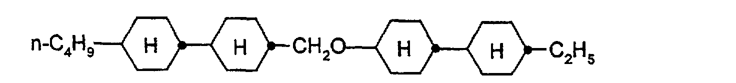

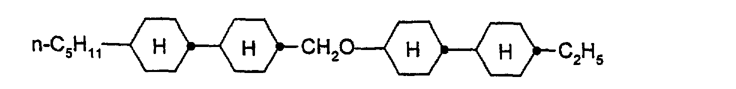

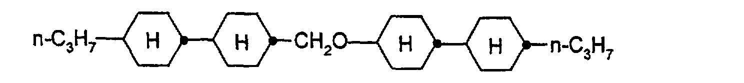

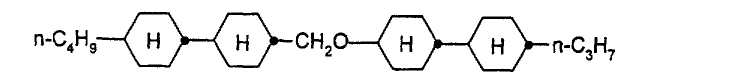

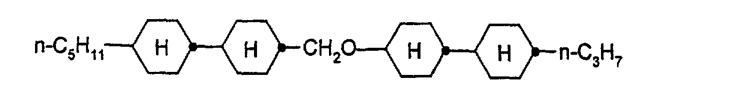

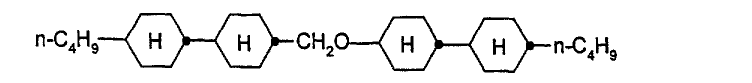

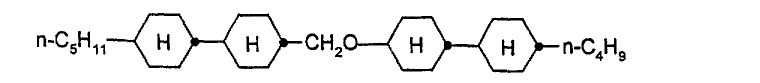

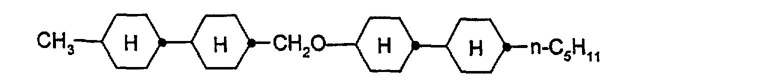

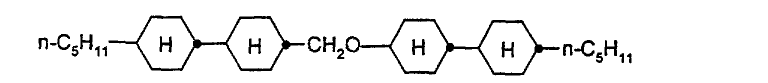

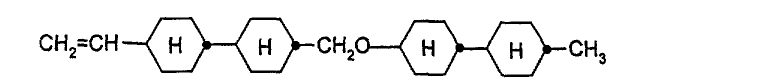

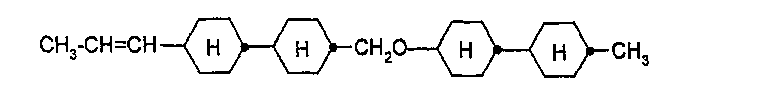

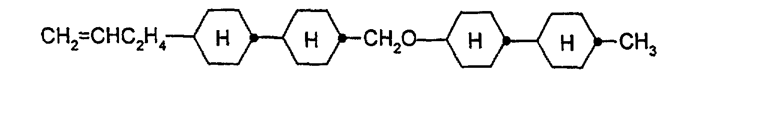

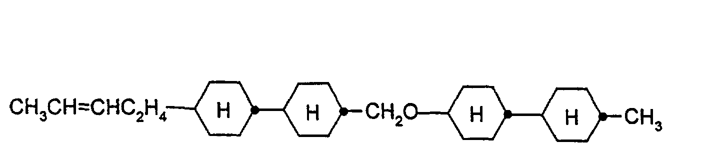

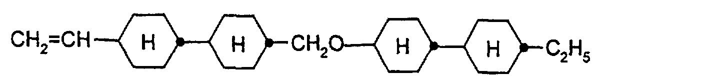

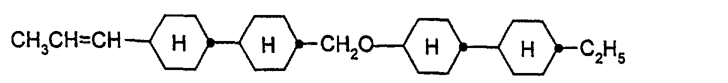

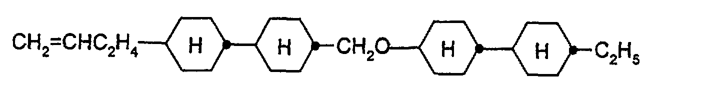

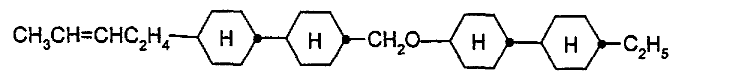

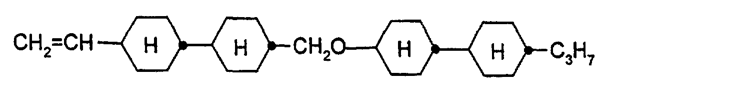

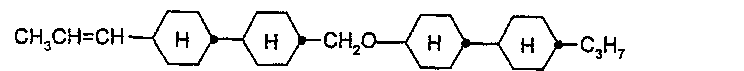

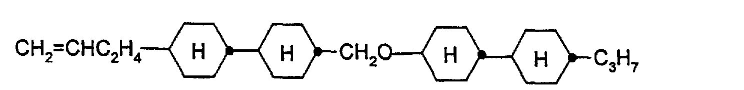

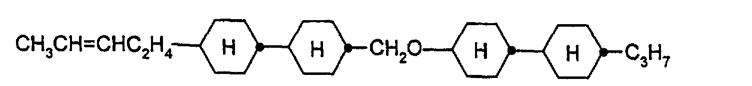

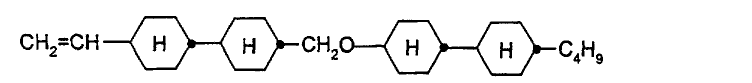

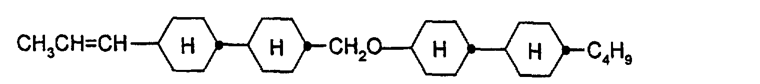

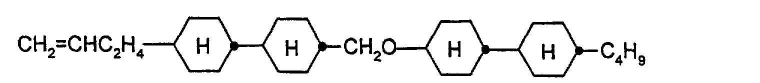

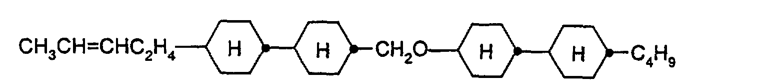

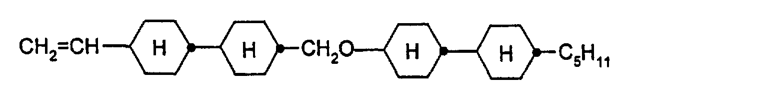

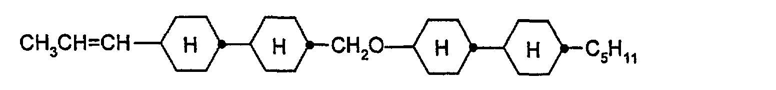

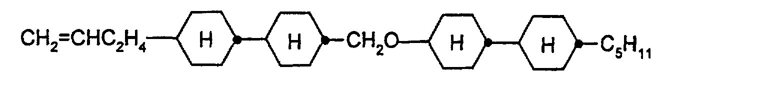

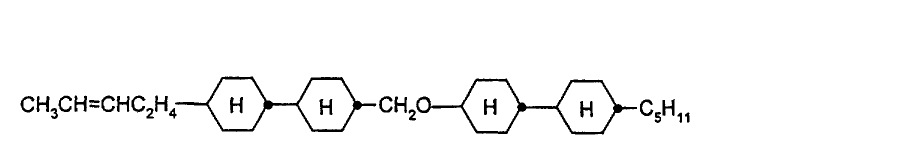

### Mischungsbeispiele

| Beispiel M1 | | | |
|---|---|---|---|
| CCH-301 | 10.00 % | S→N [°C] | < -40 |
| CCH-501 | 11.00 % | Klärpunkt [°C] | +94.5 |
| CCH-34 | 4.00 % | Δn [589 nm, 20 °C] | +0.0600 |
| CC-5-V | 15.00 % | d · Δn [20 °C] [µm] | 0.50 |
| CCP-2F.F.F | 8.00 % | Verdrillung [°] | 90 |
| CDU-2-F | 4.00 % | V₁₀ [V] | 2.26 |
| CDU-3-F | 5.00 % | | |
| CCZU-2-F | 5.00 % | | |
| CCZU-3-F | 3.00 % | | |
| CCZU-5-F | 5.00 % | | |
| CCPC-33 | 4.00 % | | |
| CCPC-34 | 5.00 % | | |
| CCPC-35 | 4.00 % | | |
| CCOC-3-3 | 3.00 % | | |
| CCOC-4-3 | 4.00 % | | |
| CCOC-3-5 | 2.00 % | | |
| CCH-5CF3 | 8.00 % | | |

| Beispiel M2 | | | |
|---|---|---|---|
| CCH-34 | 5.00 % | S→N [°C] | < -40 |
| CC-5-V | 12.00 % | Klärpunkt [°C] | +79.0 |
| CCH-5CF3 | 8.00 % | Δn [589 nm, 20 °C] | +0.0648 |
| CCP-2F.F.F | 12.00 % | d · Δn [20 °Cl [µm] | 0.50 |
| CCP-3F.F.F | 11.00 % | Verdrillung [°] | 90 |
| CCP-5F.F.F | 6.00 % | V₁₀ [V] | 1.55 |
| CCP-20CF3.F | 10.00 % | | |
| CCP-50CF3.F | 5.00 % | | |
| CCP-40CF3 | 6.00 % | | |
| CDU-2-F | 6.00 % | | |
| CDU-3-F | 10.00 % | | |
| CCOC-3-3 | 3.00 % | | |
| CCOC-4-3 | 4.00 % | | |
| CCOC-3-5 | 2.00 % | | |

| Beispiel M3 | | | |
|---|---|---|---|
| CCH-34 | 5.00 % | S→N [°C] | < -40 |
| CC-5-V | 12.00 % | Klärpunkt [°C] | +78.5 |
| CCH-5CF3 | 8.00 % | Δn [589 nm, 20 °C] | +0.0650 |
| CCP-2F.F.F | 11.00 % | d · Δn [20 °C] [µm] | 0.50 |
| CCP-3F.F.F | 12.00 % | Verdrillung [°] | 90 |
| CCP-5F.F.F | 5.00 % | V₁₀ [V] | 1.57 |
| CCP-20CF3.F | 9.00 % | | |
| CCP-50CF3.F | 5.00 % | | |
| CCP-40CF3 | 7.00 % | | |
| CDU-2-F | 6.00 % | | |
| CDU-3-F | 10.00 % | | |
| CCOC-3-3 | 3.00 % | | |
| CCOC-4-3 | 5.00 % | | |

| Beispiel M4 | | | |
|---|---|---|---|
| CCH-34 | 5.00 % | S→N [°C] | < -40 |
| CC-5-V | 6.00 % | Klärpunkt [°C]: | +80.5 |
| CCH-3CF3 | 6.00 % | Δn [589 nm, 20 °C] | +0.0644 |
| CCH-5CF3 | 8.00 % | d Δn [20 °C] [µm] | 0.50 |
| CCP-2F.F.F | 11.00 % | Verdrillung [°] | 90 |
| CCP-3F.F.F | 12.00 % | V₁₀ [V] | 1.56 |
| CCP-5F.F.F | 5.00 % | | |
| CCZU-2-F | 5.00 % | | |
| CCZU-3-F | 15.00 % | | |
| CCZU-5-F | 4.00 % | | |
| CCP-20CF3.F | 10.50 % | | |
| CCP-40CF3 | 6.50 % | | |
| CCOC-4-3 | 4.00 % | | |
| CCOC-3-3 | 2.00 % | | |

| Beispiel M5 | | | |
|---|---|---|---|
| CCH-34 | 6.00 % | S→N [°C] | < -40 |
| CCH-3CF3 | 3.00 % | Klärpunkt [°C]: | +75.0 |
| CCH-5CF3 | 8.00 % | Δn [589 nm, 20 °C] | +0.644 |
| CCP-2F.F.F | 11.00 % | Δε [1kHz, 20 °C] | +10.1 |
| CCP-3F.F.F | 10.00 % | d · Δn [20 °C] [µm] | 0.50 |
| CCP-5F.F.F | 6.00 % | Verdrillung [°] | 90 |
| CCP-20CF3.F | 4.00 % | V₁₀ [V] | 1.34 |
| CCP-40CF3 8.00 | % | | |
| CDU-2-F | 10.00 % | | |
| CDU-3-F | 12.00 % | | |
| CDU-5-F | 10.00 % | | |
| CCOC-3-3 | 4.00 % | | |
| CCOC-4-3 | 8.00 % | | |

| Beispiel M6 | | | |
|---|---|---|---|
| CCH-34 | 5.00 % | Klärpunkt [°C] | +80.0 |
| CC-5-V | 8.00 % | Δn [589 nm, 20 °C] | +0.0642 |
| CCH-3CF3 | 6.00 % | Δε [1kHz, 20 °C] | +7.8 |
| CCH-5CF3 | 8.00 % | d · Δn [20 °C] [µm] | 0.50 |
| CCP-2F.F.F | 11.00 % | Verdrillung [°] | 90 |
| CCP-3F.F.F | 11.00 % | V₁₀ [V] | 1.58 |
| CCP-5F.F.F | 6.00 % | | |
| CCZU-2-F | 6.00 % | | |
| CCZU-3-F | 14.00 % | | |
| CCZU-5-F | 6.00 % | | |
| CCP-20CF3.F | 8.00 % | | |
| CCP-40CF3 | 4.00 % | | |
| CCOC-3-3 | 5.00 % | | |
| CCOC-4-3 | 2.00 % | | |

| Beispiel M7 | | | |
|---|---|---|---|
| CCH-34 | 6.00 % | Klärpunkt [°C] | +79.5 |
| CC-5-V | 14.00 % | Δn [589 nm, 20 °C] | +0.0649 |
| CCP-2F.F.F | 11.00 % | Δε [1kHz, 20 °C] | +9.5 |
| CCP-3F.F.F | 11.00 % | d · Δn [20 °C] [µm] | 0.50 |
| CCP-5F.F.F | 6.00 % | Verdrillung [°] | 90 |
| CCP-20CF3.F | 6.00 % | V₁₀ [V] | 1.38 |
| CDU-2-F | 10.00 % | | |
| CDU-3-F | 14.00 % | | |
| CDU-5-F | 10.00 % | | |
| CCOC-3-3 | 4.00 % | | |
| CCOC-4-3 | 8.00 % | | |

| Beispiel M8 | |
|---|---|
| CCH-34 | 6.00 % |
| CCH-501 | 8.00 % |
| CCP-2F.F.F | 11.00 % |
| CCP-3F.F.F | 11.00 % |
| CCP-5F.F.F | 6.00 % |
| CCP-20CF3.F | 4.00 % |
| CCP-40CF3 | 4.00 % |
| CDU-2-F | 12.00 % |
| CDU-3-F | 16.00 % |
| CDU-5-F | 8.00 % |
| CCOC-3-3 | 4.00 % |
| CCOC-4-3 | 8.00 % |

| Beispiel M9 | | | |
|---|---|---|---|
| CCH-34 | 5.00 % | S→N [°C] | < -40 |
| CC-5-V | 8.00 % | Klärpunkt [°C] | +80.5 |
| CCH-3CF3 | 6.00 % | Δn [589 nm, 20 °C] | +0.0643 |
| CCH-5CF3 | 8.00 % | Δε [1kHz, 20 °C] | +7.8 |
| CCP-2F.F.F | 11.00 % | d · Δn [20 °C] [µm] | 0.50 |
| CCP-3F.F.F | 11.00 % | Verdrillung [°] | 90 |
| CCP-5F.F.F | 6.00 % | V₁₀ [V] | 1.59 |
| CCZU-2-F | 5.00 % | | |
| CCZU-3-F | 15.00 % | | |
| CCZU-5-F | 5.00 % | | |
| CCP-20CF3.F | 8.00 % | | |
| CCP-40CF3 | 5.00 % | | |
| CCOC-4-3 | 5.00 % | | |
| CCOC-3-3 | 2.00 % | | |

| Beispiel M10 | | | |
|---|---|---|---|
| CCH-34 | 5.00 % | S→N [°C] | < -40 |
| CC-5-V | 6.00 % | Klärpunkt [°C] | +80.0 |
| CCH-3CF3 | 6.00 % | Δn [589 nm, 20 °C] | +0.0848 |
| CCH-5CF3 | 6.00 % | Δε [1kHz, 20 °C] | +8.0 |
| CCP-2F.F.F | 12.00 % | d · Δn [20 °C] [µm] | 0.50 |
| CCP-3F.F.F | 11.00 % | Verdrillung [°] | 90 |
| CCP-5F.F.F | 6.00 % | V₁₀ [V] | 1.54 |
| CCP-20CF3.F | 8.00 % | | |
| CCP-50CF3.F | 8.00 % | | |
| CCP-40CF3 | 6.00 % | | |
| CDU-2-F | 6.00 % | | |
| CDU-3-F | 8.00 % | | |
| CCOC-3-3 | 4.00 % | | |
| CCOC-4-3 | 8.00 % | | |

| Beispiel M11 | | | |
|---|---|---|---|
| CCH-34 | 5.00 % | Klärpunkt [°C] | +80.0 |
| CC-5-V | 12.00 % | Δn [589 nm, 20 °C] | +0.0644 |
| CCH-3CF3 | 5.00 % | d · Δn [20 °C] [µm] | 0.50 |
| CCH-5CF3 | 8.00 % | Verdrillung [°] | 90 |
| CCP-2F.F.F | 12.00 % | V₁₀ [V] | 1.63 |
| CCP-3F.F.F | 11.00 % | | |
| CCP-5F.F.F | 6.00 % | | |
| CCP-20CF3.F | 8.00 % | | |
| CCP-50CF3.F | 3.00 % | | |
| CCP-40CF3 | 8.00 % | | |
| DCZG-2-OT | 4.00 % | | |
| DCZG-3-OT | 4.00 % | | |
| DCZG-5-OT | 6.00 % | | |
| CCOC-3-3 | 2.00 % | | |
| CCOC-4-3 | 6.00 % | | |

| Beispiel M12 | | | |
|---|---|---|---|
| CCH-34 | 5.00 % | S→N [°C] | < -40.0 |
| CC-5-V | 6.00 % | Klärpunkt [°C] | +80.5 |
| CCH-3CF3 | 6.00 % | Δn [589 nm, 20 °C] | +0.0654 |
| CCH-5CF3 | 8.00 % | d · Δn [20 °C] [µm] | 0.50 |
| CCP-2F.F.F | 12.00 % | Verdrillung [°] | 90 |
| CCP-3F.F.F | 11.00 % | V₁₀ [V] | 1.53 |
| CCP-5F.F.F | 6.00 % | | |
| CCZU-2-F | 5.00 % | | |
| CCZU-3-F | 14.00 % | | |
| CCZU-5-F | 5.00 % | | |
| CCP-20CF3.F | 9.00 % | | |
| CCP-40CF3 | 7.00 % | | |
| CCOC-3-3 | 4.00 % | | |
| CCOC-4-3 | 2.00 % | | |

| Beispiel M13 | | | |
|---|---|---|---|
| CCH-34 | 4.00 % | S→N [°C] | < -40 |
| CC-3-DT | 12.00 % | Klärpunkt [°C] | +81.0 |
| CCH-5CF3 | 8.00 % | Δn [589 nm, 20 °C] | +0.0647 |
| CCP-2F.F.F | 12.00 % | d · Δn [20 °C] [µm] | 0.50 |
| CCP-3F.F.F | 11.00 % | Verdrillung [°] | 90 |
| CCP-5F.F.F | 6.00 % | V₁₀ [V] | 1.53 |
| CCZU-2-F | 4.00 % | | |
| CCZU-3-F | 10.00 % | | |
| CCZU-5-F | 4.00 % | | |
| CCP-20CF3.F | 8.00 % | | |
| CCP-50CF3.F | 6.00 % | | |
| CCP-40CF3 | 7.00 % | | |
| CCOC-4-3 | 5.00 % | | |
| CCOC-3-3 | 3.00 % | | |

| Beispiel M14 | |
|---|---|
| CCH-34 | 5.00 % |
| CCH-35 | 4.00 % |
| CC-3-V1 | 12.00 % |
| CC-5-V | 17.00 % |
| CCH-3CF3 | 4.00 % |
| CCH-5CF3 | 8.00 % |
| CCP-2F.F.F | 10.00 % |
| CCZU-2-F | 5.00 % |
| CCZU-3-F | 12.00 % |
| CCZU-5-F | 5.00 % |
| CCOC-3-3 | 3.00 % |
| CCOC-4-3 | 4.00 % |
| CCOC-3-5 | 2.00 % |
| CH-43 | 3.00 % |
| CH-33 | 3.00 % |
| CH-35 | 3.00 % |

| Beispiel M15 | |
|---|---|
| CCH-34 | 5.00 % |
| CC-3-V1 | 13.00 % |
| CC-5-V | 14.00 % |
| CC-3-DT | 13.00 % |
| CCH-5CF3 | 6.00 % |
| CCP-2F.F.F | 10.00 % |
| CCP-5F.F.F | 3.00 % |
| CCP-40CF3 | 4.00 % |
| CCZU-2-F | 5.00 % |
| CCZU-3-F | 6.00 % |
| CCZU-5-F | 5.00 % |
| CCOC-3-3 | 3.00 % |
| CCOC-4-3 | 4.00 % |
| CH-43 | 3.00 % |
| CH-33 | 3.00 % |
| CH-35 | 3.00 % |

| Beispiel M16 | |
|---|---|
| CCH-34 | 5.00 % |
| CC-3-V1 | 12.00 % |
| CC-5-V | 17.00 % |
| CCH-3CF3 | 8.00 % |
| CCH-5CF3 | 8.00 % |
| CCP-2F.F.F | 11.00 % |
| CCP-5F.F.F | 3.00 % |
| CCP-40CF3 | 2.00 % |
| CCZU-2-F | 5.00 % |
| CCZU-3-F | 7.00 % |
| CCZU-5-F | 6.00 % |
| CCOC-3-3 | 3.00 % |
| CCOC-4-3 | 4.00 % |
| CH-43 | 3.00 % |
| CH-33 | 3.00 % |
| CH-35 | 3.00 % |

| Beispiel M17 | | | |
|---|---|---|---|
| CCH-34 | 5.00 % | S→N [°C] | < -40 |
| CC-5-V | 12.00 % | Klärpunkt [°C] | +79.0 |
| CCH-5CF3 | 8.00 % | Δn [589 nm, 20 °C] | +0.0648 |
| CCP-2F.F.F | 12.00 % | d · Δn [20 °C] [µm] | 0.50 |
| CCP-3F.F.F | 11.00 % | Verdrillung [°] | 90 |
| CCP-5F.F.F | 6.00 % | V₁₀ [V] | 1.55 |
| CCP-20CF3.F | 10.00 % | | |
| CCP-50CF3.F | 5.00 % | | |
| CCP-40CF3 | 6.00 % | | |
| CDU-2-F | 6.00 % | | |
| CDU-3-F | 10.00 % | | |
| CCOC-3-3 | 3.00 % | | |
| CCOC-4-3 | 4.00 % | | |
| CCOC-4-5 | 2.00 % | | |

| Beispiel M18 | | | |
|---|---|---|---|
| CCH-34 | 5.00 % | S→N [°C] | < -40 |
| CC-5-V | 11.00 % | Klärpunkt [°C] | +79.5 |
| CCH-5CF3 | 8.00 % | Δn [589 nm, 20 °C] | +0.0653 |
| CCP-2F.F.F | 12.00 % | d · Δn [20 °C] [µm] | 0.50 |
| CCP-3F.F.F | 11.50 % | Verdrillung [°] | 90 |
| CCP-5F.F.F | 5.50 % | V₁₀ [V] | 1.54 |
| CCP-20CF3.F | 11.00 % | | |
| CCP-50CF3.F | 7.00 % | | |
| CCP-40CF3 | 5.00 % | | |
| CDU-2-F | 7.00 % | | |
| CDU-3-F | 8.00 % | | |
| CCOC-3-3 | 3.00 % | | |
| CCOC-4-3 | 4.00 % | | |
| CCOC-4-5 | 2.00 % | | |

| Beispiel M19 | | | |
|---|---|---|---|
| CCH-34 | 5.00% | S→N [°C] | < -40 |
| CC-5-V 6.00 % | | Klärpunkt [°C] | +80.5 |
| CCH-3CF3 | 6.00 % | Δn [589 nm, 20 °C] | +0,0644 |
| CCH-5CF3 | 8.00 % | d · Δn [20 °C] [µm] | 0.50 |
| CCP-2F.F.F | 11.00 % | Verdrillung [°] | 90 |
| CCP-3F.F.F | 12.00 % | V₁₀ [V] | 1.56 |
| CCP-5F.F.F | 5.00 % | | |
| CCZU-2-F | 5.00 % | | |
| CCZU-3-F | 15.00 % | | |
| CCZU-5-F | 4.00 % | | |
| CCP-20CF3.F | 10.50 % | | |
| CCP-40CF3 | 6.50 % | | |
| CCOC-4-3 | 4.00 % | | |
| CCOC-3-3 | 2.00 % | | |

| Beispiel M20 | |
|---|---|
| CCH-301 | 6.00 % |
| CCH-302 | 12.00 % |
| CCH-501 | 18.00 % |
| CC-5-V | 4.00 % |
| CCP-20CF3.F | 7.00 % |
| CCP-50CF3.F | 5.00 % |
| CCP-40CF3 | 7.00 % |
| CCZU-2-F | 5.00 % |
| CCZU-3-F | 15.00 % |
| CCZU-5-F | 5.00 % |
| CCOC-3-3 | 6.00 % |
| CCOC-4-3 | 6.00 % |
| CCOC-3-5 | 4.00 % |

| Beispiel M21 | |
|---|---|
| CCH-302 | 12.00 % |
| CCH-501 | 18.00 % |
| CC-5-V | 5.00 % |
| CCH-34 | 5.00 % |
| CCP-3F.F.F | 4.00 % |
| CCP-20CF3.F | 8.00 % |
| CCP-20CF3 | 2.00 % |
| CCP-40CF3 | 6.00 % |
| CCZU-2-F | 5.00 % |
| CC2U-3-F | 15.00 % |
| CCZU-5-F | 4.00 % |
| CCOC-3-3 | 6.00 % |
| CCOC-4-3 | 6.00 % |
| CCOC-3-5 | 4.00 % |

| Beispiel M22 | | | |
|---|---|---|---|
| CCH-301 | 20.00 % | S→N [°C] | < -40 |
| CCH-501 | 15.50 % | Klärpunkt [°C] | +93.5 |
| CC-5-V | 11.50 % | Δn [589 nm, 20 °C] | +0.0605 |
| CDU-2-F | 6.00 % | d · Δn [20 °C] [µm] | 0.50 |
| CDU-3-F | 6.00 % | Verdrillung [°] | 90 |
| CDU-5-F | 3.00 % | V₁₀ [V] | 2.18 |
| CCZU-2-F | 3.00 % | | |
| CCZU-3-F | 11.00 % | | |
| CCZU-5-F | 3.00 % | | |
| CCPC-33 | 5.00 % | | |
| CCPC-34 | 4.00 % | | |
| CCPC-35 | 4.00 % | | |
| CCOC-3-3 | 3.00 % | | |
| CCOC-4-3 | 3.00 % | | |
| CCOC-3-5 | 2.00 % | | |

| Beispiel M23 | | | |
|---|---|---|---|
| CCH-301 | 20.00 % | S→N [°C] | < -40 |
| CCH-501 | 14.00 % | Klärpunkt [°C] | +94.5 |
| CCH-34 | 3.00 % | Δn [589 nm, 20 °C] | +0.0604 |
| CC-5-V | 10.00 % | d · Δn [20 °C] [µm] | 0.50 |
| CCP-2F.F.F | 4.00 % | Verdrillung [°] | 90 |
| CDU-2-F | 3.00 % | V₁₀ [V] | 2.21 |
| CDU-3-F | 7.00 % | | |
| CDU-5-F | 3.00 % | | |
| CCZU-2-F | 3.00 % | | |
| CCZU-3-F | 10.00 % | | |
| CCZU-5-F | 2.00 % | | |
| CCPC-33 | 5.00 % | | |
| CCPC-34 | 4.00 % | | |
| CCPC-35 | 4.00 % | | |
| CCOC-3-3 | 2.00 % | | |
| CCOC-4-3 | 4.00 % | | |
| CCOC-3-5 | 2.00 % | | |

| Beispiel M24 | | | |
|---|---|---|---|
| CCH-301 | 14.00 % | S→N [°C] | < -40 |
| CCH-501 | 14.00 % | Klärpunkt [°C] | +97.5 |
| CCH-34 | 4.00 % | Δn [589 nm, 20 °C] | +0.0616 |
| CC-5-V | 12.00 % | d · Δn [20 °C] [µm] | 0.50 |
| CCP-2F.F.F | 8.00 % | Verdrillung [°] | 90 |
| CCP-3F.F.F | 2.00 % | V₁₀ [V] | 2.19 |
| CCP-5F.F.F | 3.00 % | | |
| CDU-2-F | 4.00 % | | |
| CDU-3-F | 6.00 % | | |
| CCZU-2-F | 3.00 % | | |
| CCZU-3-F | 9.00 % | | |
| CCPC-33 | 4.00 % | | |
| CCPC-34 | 4.00 % | | |
| CCPC-35 4.00 | % | | |
| CCOC-3-3 | 3.00 % | | |
| CCOC-4-3 | 4.00 % | | |
| CCOC-3-5 | 2.00 % | | |

| Beispiel M25 | | | |
|---|---|---|---|
| CCH-301 | 10.00 % | S→N [°C] | < -40 |
| CCH-501 | 14.00 % | Klärpunkt [°C] | +95.5 |
| CCH-34 | 5.00 % | Δn [589 nm, 20 °C] | +0.0609 |
| CC-5-V | 9.00 % | d · Δn [20 °C] [µm] | 0.50 |
| CCH-5CF3 | 6.00 % | Verdrillung [°] | 90 |
| CCP-2F.F.F | 9.00 % | V₁₀ [V] | 2.22 |
| CCP-3F.F.F | 5.00 % | | |
| CCP-5F.F.F | 4.00 % | | |
| CDU-3-F | 7.00 % | | |
| CCZU-2-F | 3.00 % | | |
| CCZU-3-F | 6.00 % | | |
| CCPC-33 | 4.00 % | | |
| CCPC-34 | 4.00 % | | |
| CCPC-35 | 4.00 % | | |
| CCOC-3-3 | 3.00 % | | |
| CCOC-4-3 | 5.00 % | | |
| CCOC-3-5 | 2.00 % | | |

| Beispiel M26 | | | |
|---|---|---|---|
| CCH-301 | 10.00% | S→N [°C] | < -40 |
| CCH-501 | 11.00 % | Klärpunkt [°C] | +94.5 |
| CCH-34 | 4.00 % | Δn [589 nm, 20 °C] | +0.0600 |
| CC-5-V | 15.00 % | d · Δn [20 °C] [um] | 0.50 |
| CCP-2F.F.F | 8.00 % | Verdrillung [°] | 90 |
| CDU-2-F | 4.00 % | V₁₀ [V] | 2.26 |
| CDU-3-F | 5.00 % | | |
| CCZU-2-F | 5.00 % | | |
| CCZU-3-F | 3.00 % | | |
| CCZU-5-F | 5.00 % | | |
| CCPC-33 | 4.00 % | | |
| CCPC-34 | 5.00 % | | |
| CCPC-35 | 4.00 % | | |
| CCOC-3-3 | 3.00 % | | |
| CCOC-4-3 | 4.00 % | | |
| CCOC-4-5 | 2.00 % | | |
| CCH-5CF3 | 8.00 % | | |

| Beispiel M27 | | | |
|---|---|---|---|
| CCH-34 | 5.00 % | S→N [°C] | < -40 |
| CC-5-V | 10.00 % | Klärpunkt [°C] | +78.5 |
| CCH-301 | 8.00 % | Δn [589 nm, 20 °C] | +0.0655 |
| CCP-2F.F.F | 11.00 % | d · Δn [20 °C] [µm] | 0.50 |
| CCP-3F.F.F | 12.00 % | Verdrillung [°] | 90 |
| CCP-5F.F.F | 5.00 % | V₁₀ [V] | 1.51 |
| CCP-20CF3.F | 10.00 % | | |
| CCP-50CF3.F | 6.00 % | | |
| CCP-40CF3 | 6.00 % | | |
| CDU-2-F | 9.00 % | | |
| CDU-3-F | 10.00 % | | |
| CCOC-3-3 | 3.00 % | | |
| CCOC-4-3 | 5.00 % | | |

| Beispiel M28 | | | |
|---|---|---|---|
| CCH-34 | 5.00 % | S→N [°C] | < -40 |
| CC-5-V | 14.00 % | Klärpunkt [°C] | +78.5 |
| CCH-5CF3 | 8.00 % | Δn [589 nm, 20 °C] | +0.0650 |
| CCP-2F.F.F | 11.00 % | d · Δn [20 °C] [µm] | 0.50 |
| CCP-3F.F.F | 12.00 % | Verdrillung [°] | 90 |
| CCP-5F.F.F | 5.00 % | V₁₀ [V] | 1.57 |
| CCP-20CF3.F | 9.00 % | | |
| CCP-50CF3.F | 5.00 % | | |
| CCP-40CF3 | 7.00 % | | |
| CDU-2-F | 6.00 % | | |
| CDU-3-F | 10.00 % | | |
| CCOC-3-3 | 3.00 % | | |
| CCOC-4-3 | 5.00 % | | |

| Beispiel M29 | |
|---|---|
| CCH-34 | 5.00 % |
| CC-5-V | 12.00 % |
| CCH-5CF3 | 4.00 % |
| CCH-3CF3 | 3.00 % |
| CCP-2F.F.F | 12.00 % |
| CCP-3F.F.F | 11.00 % |
| CCP-5F.F.F | 7.00 % |
| CCP-20CF3.F | 10.00 % |
| CCP-50CF3.F | 5.00 % |
| CCP-40CF3 | 6.00 % |
| CDU-2-F | 6.00 % |
| CDU-3-F | 10.00 % |
| CCOC-3-3 | 4.00 % |
| CCOC-4-3 | 5.00 % |

| Beispiel M30 | |
|---|---|
| CCH-35 | 5.00 % |
| CC-5-V | 12.00 % |
| CCH-5CF3 | 4.00 % |
| CCH-3CF3 | 3.00 % |
| CCP-2F.F.F | 12.00 % |
| CCP-3F.F.F | 11.00 % |
| CCP-5F.F.F | 7.00 % |
| CCP-20CF3.F | 10.00 % |
| CCP-50CF3.F | 5.00 % |
| CCP-40CF3 | 6.00 % |
| CDU-2-F | 6.00 % |
| CDU-3-F | 10.00 % |
| CCOC-3-3 | 4.00 % |
| CCOC-4-3 | 5.00 % |

| Beispiel M31 | | | |
|---|---|---|---|
| CCH-35 | 5.00 % | Klärpunkt [°C] | +78.5 |
| CC-5-V | 14.00 % | Δn [589 nm, 20 °C] | +0.0654 |
| CCH-5CF3 | 8.00 % | | |
| CCP-2F.F.F | 11.00 % | | |
| CCP-3F.F.F | 12.00 % | | |
| CCP-5F.F.F | 5.00 % | | |
| CCP-20CF3.F | 9.00 % | | |
| CCP-50CF3.F | 5.00 % | | |
| CCP-40CF3 | 7.00 % | | |
| CDU-2-F | 6.00 % | | |
| CDU-3-F | 10.00 % | | |
| CCOC-3-3 | 3.00 % | | |
| CCOC-4-3 | 5.00 % | | |

| Beispiel M32 | | | |
|---|---|---|---|
| CCH-301 | 14.00 % | Klärpunkt [°C] | +95.0 |
| CCH-501 | 14.00 % | Δn [589 nm, 20 °C] | +0.0611 |
| CCH-35 | 5.00 % | d · Δn [20 °C] [µm] | 0.50 |
| CC-5-V | 11.00 % | Verdrillung [°] | 90 |
| CCP-2F.F.F | 9.00 % | V₁₀ [V] | 2.22 |
| CCP-3F.F.F | 4.00 % | | |
| CCP-5F.F.F | 5.00 % | | |
| CDU-2-F | 6.00 % | | |
| CDU-3-F | 9.00 % | | |
| CCPC-33 | 5.00 % | | |
| CCPC-34 | 4.00 % | | |
| CCPC-35 | 4.00 % | | |
| CCOC-3-3 | 3.00 % | | |
| CCOC-4-3 | 5.00 % | | |
| CCOC-3-5 | 2.00 % | | |

| Beispiel M33 | | | |
|---|---|---|---|
| CCH-301 | 14.00 % | Klärpunkt [°C] | 94.5 |
| CCH-501 | 14.00 % | Δn [589 nm, 20 °C] | +0.0608 |
| CCH-35 | 5.00 % | d · Δn [20 °C] [µm] | 0.50 |
| CC-5-V | 11.00 % | Verdrillung [°] | 90 |
| CCP-2F.F.F | 11.00 % | V₁₀ [V] | 2.20 |
| CCP-5F.F.F | 6.00 % | | |
| CDU-2-F | 6.00 % | | |
| CDU-3-F | 10.00 % | | |
| CCPC-33 | 4.00 % | | |
| CCPC-34 | 4.00 % | | |
| CCPC-35 | 5.00 % | | |
| CCOC-3-3 | 3.00 % | | |
| CCOC-4-3 | 5.00 % | | |
| CCOC-3-5 | 2.00 % | | |

| Beispiel M34 | | | |
|---|---|---|---|
| CCH-301 | 16.00 % | S→N [°C] | < -40 |
| CCH-501 | 16.00 % | Klärpunkt [°C] | 95.5 |
| CCH-35 | 3.00 % | Δn [589 nm, 20 °C] | +0.0608 |
| CCH-5CF3 | 5.00 % | Δε [1 kHz, 20 °C] | +4.5 |
| CCP-2F.F.F | 10.00 % | d · Δn [20 °C] [µm] | 0.50 |
| CCP-5F.F.F | 8.00% | Verdrillung [°] | 90 |
| CCZU-2-F | 4.00 % | V₁₀ [V] | 2.13 |
| CCZU-3-F | 13.00 % | | |
| CCZU-5-F | 4.00 % | | |
| CCPC-33 | 3.00 % | | |
| CCPC-34 | 4.00 % | | |
| CCPC-35 | 4.00 % | | |
| CCOC-3-3 | 3.00 % | | |
| CCOC-4-3 | 5.00 % | | |
| CCOC-3-5 | 2.00 % | | |

| Beispiel M35 | | | |
|---|---|---|---|
| CCH-3CF3 | 7.00 % | Klärpunkt [°C] | +80.0 |
| CCH-301 | 6.00 % | Δn [589 nm, 20 °C] | +0.0689 |
| CCH-501 | 5.00 % | d · Δn [20 °C] [µm] | 0.50 |
| CCP-2F.F.F | 10.00 % | Verdrillung [°] | 90 |
| CCP-3F.F.F | 13.00 % | V₁₀ [V] | 1.40 |
| CCP-5F.F.F | 5.00 % | | |
| CCZU-3-F | 13.00 % | | |
| CCZU-5-F | 7.00 % | | |
| CCP-50CF2.F.F | 8.00 % | | |
| CDU-3-F | 9.00 % | | |
| CCPC-30CF3.F | 7.00 % | | |
| CCPC-50CF3.F | 7.00 % | | |
| CCOC-3-3 | 3.00 % | | |

| Beispiel M36 | | | |
|---|---|---|---|
| CCH-3CF3 | 8.00 % | Klärpunkt [°C]: | +81.0 |
| CCH-5CF3 | 5.00 % | Δn [589 nm, 20 °C] | +0.0655 |
| CCH-301 | 9.00 % | Δε [1kHz, 20 °C] | +8.7 |
| CCP-2F.F.F | 8.00 % | d · Δn [20 °C] [µm] | 0.50 |
| CCP-3F.F.F | 13.00 % | Verdrillung [°] | 90 |
| CCP-5F.F.F | 5.00 % | V₁₀ [V] | 1.47 |
| CCZU-2-F | 5.00 % | | |
| CCZU-3-F | 8.00 % | | |
| CCZU-5-F | 5.00 % | | |
| CCP-30CF3.F | 8.00 % | | |
| CCP-50CF2.F.F | 8.00 % | | |
| CDU-3-F | 9.00 % | | |
| CCOC-3-3 | 5.00 % | | |
| CPCC-2-3 | 4.00 % | | |

| Beispiel M37 | |
|---|---|
| CCH-3CF3 | 8.00 % |
| CCH-5CF3 | 5.00 % |
| CCH-301 | 8.00 % |
| CCP-2F.F.F | 8.00 % |
| CCP-3F.F.F | 13.00 % |
| CCP-5F.F.F | 5.00 % |
| CCZU-2-F | 7.00 % |
| CCZU-3-F | 15.00 % |
| CCZU-5-F | 7.00 % |
| CCP-50CF2.F.F | 4.00 % |
| CDU-3-F | 10.00 % |
| CCOC-3-3 | 6.00 % |
| CPCC-2-3 | 4.00 % |

| Beispiel M38 | | | |
|---|---|---|---|
| CCH-3CF3 | 8.00 % | Klärpunkt [°C] | +77.0 |
| CCH-5CF3 | 5.00 % | Δn [589 nm, 20 °C] | +0.0663 |
| CCH-301 | 8.00 % | d · Δn [20 °C] [µm] | 0.50 |
| CCP-2F.F.F | 4.00 % | Verdrillung [°] | 90 |
| CCP-3F.F.F | 13.00 % | V₁₀ [V] | 1.38 |
| CCP-5F.F.F | 5.00 % | | |
| CCZU-3-F | 8.00 % | | |
| CCZU-5-F | 5.00 % | | |
| CCP-30CF3.F | 8.00 % | | |
| CCP-50CF2.F.F | 8.00 % | | |
| CDU-2-F | 9.00 % | | |
| CDU-3-F | 10.00 % | | |
| CCOC-3-3 | 5.00 % | | |
| CPCC-2-3 | 4.00 % | | |

| Beispiel M39 | | | |
|---|---|---|---|
| CCH-3CF3 | 11.00 % | Klärpunkt [°C] | +79.5 |
| CCH-5CF3 | 7.00 % | Δn [589 nm, 20 °C] | +0.0656 |
| CCP-2F.F.F | 4.00 % | d · Δn [20 °C] [µm] | 0.50 |
| CCP-3F.F.F | 10.00 % | Verdrillung [°] | 90 |
| CCP-5F.F.F | 5.00 % | V₁₀ [V] | 1.38 |
| CCZU-3-F | 9.00 % | | |
| CCZU-5-F | 5.00 % | | |
| CCP-30CF3.F | 8.00 % | | |
| CCP-50CF2.F.F | 5.00 % | | |
| CDU-2-F | 9.00 % | | |
| CDU-3-F | 10.00 % | | |
| CDU-5-F | 5.00 % | | |
| CCOC-3-3 | 8.00 % | | |
| CPCC-2-3 | 4.00 % | | |

| Beispiel M40 | | | |
|---|---|---|---|
| CCH-3CF3 | 11.00 % | Klärpunkt [°C] | +79.5 |
| CCH-5CF3 | 9.00 % | Δn [589 nm, 20 °C] | +0.0670 |
| CCP-2F.F.F | 4.00 % | d · Δn [20 °C] [um] | 0.50 |
| CCP-3F.F.F | 10.00 % | Verdrillung [°] | 90 |
| CCP-5F.F.F | 5.00 % | V₁₀ [V] | 1.36 |
| CCP-30CF3.F | 8.00 % | | |
| CCP-50CF2.F.F | 5.00 % | | |
| CDU-2-F | 9.00 % | | |
| CDU-3-F | 10.00 % | | |
| CDU-5-F | 5.00 % | | |
| CCOC-3-3 | 7.00 % | | |
| CPCC-2-3 | 8.00 % | | |
| DCZU-3-F | 9.00 % | | |

| Beispiel M41 | |
|---|---|
| CCH-3CF3 | 7.00 % |
| CCH-5CF3 | 7.00 % |
| CCP-2F.F.F | 4.00 % |
| CCP-3F.F.F | 10.00 % |
| CCP-5F.F.F | 5.00 % |
| CCZU-3-F | 9.00 % |
| CCZU-5-F | 5.00 % |
| CCP-30CF3.F | 8.00 % |
| CCP-50CF2.F.F | 5.00 % |
| CDU-2-F | 9.00 % |
| CDU-3-F | 12.00 % |
| CDU-5-F | 5.00 % |
| CCOC-3-3 | 8.00 % |
| CCOC-3-5 | 6.00 % |

| Beispiel M42 | |
|---|---|
| CCH-3CF3 | 6.00 % |
| CCH-34 | 6.00 % |
| CCP-2F.F.F | 11.00% |
| CCP-3F.F.F | 11.00% |
| CCP-5F.F.F | 6.00 % |
| CCP-20CF3.F | 12.00 % |
| CCP-50CF3.F | 7.00 % |
| CDU-2-F | 11.00 % |
| CDU-3-F | 12.00 % |
| CDU-5-F | 10.00 % |
| CCOC-3-3 | 8.00 % |

| Beispiel M43 | |
|---|---|
| CCH-3CF3 | 11.00 % |
| CCH-5CF3 | 6.00 % |
| CCP-2F.F.F | 4.00 % |
| CCP-3F.F.F | 10.00 % |
| CCP-5F.F.F | 5.00 % |
| CCZU-3-F | 9.00 % |
| CCZU-5-F | 5.00 % |
| CCP-30CF3.F | 8.00 % |
| CCP-50CF2.F.F | 5.00 % |
| CDU-2-F | 9.00 % |
| CDU-3-F | 10.00 % |
| CDU-5-F | 5.00 % |
| CCOC-3-3 | 8.00 % |
| DCC-3-5 | 5.00 % |

| Beispiel M44 | |
|---|---|
| CCH-5CF3 | 4.00 % |
| CCH-34 | 6.00 % |
| CCP-2F.F.F | 11.00 % |
| CCP-3F.F.F | 11.00 % |
| CCP-5F.F.F | 6.00 % |
| CCP-20CF3.F | 14.00 % |
| CCP-50CF3.F | 9.00 % |
| CDU-2-F | 11.00 % |
| CDU-3-F | 12.00 % |
| CDU-5-F | 8.00 % |
| CCOC-3-3 | 8.00 % |

| Beispiel M45 | | | |
|---|---|---|---|
| CCH-5CF3 | 7.00 % | S→N [°C] | < -40 |
| CCH-34 | 6.00 % | Klärpunkt [°C] | +74.5 |
| CCP-2F.F.F | 11.00 % | Δn [589 nm, 20 °C] | +0.0670 |
| CCP-3F.F.F | 11.00 % | d · Δn [20 °C] [µm] | 0.50 |
| CCP-5F.F.F | 6.00 % | Verdrillung [°] | 90 |
| CCP-20CF3.F | 12.00 % | V₁₀ [V] | 1.31 |
| CCP-40CF3 | 8.00 % | | |
| CDU-2-F | 11.00 % | | |
| CDU-3-F | 12.00 % | | |
| CDU-5-F | 8.00 % | | |
| CCOC-3-3 | 8.00 % | | |

| Beispiel M46 | |
|---|---|
| CCH-5CF3 | 7.00 % |
| CCH-34 | 6.00 % |
| CCP-2F.F.F | 11.00 11.00 % |
| CCP-3F.F.F | 11.00 % |
| CCP-5F.F.F | 6.00 % |
| CCP-20CF3.F | 6.00 % |
| CCP-40CF3 | 8.00 % |
| CDU-2-F | 11.00 % |
| CDU-3-F | 12.00 % |
| CDU-5-F | 8.00 % |
| CCOC-3-3 | 8.00 % |
| CCOC-3-5 | 6.00 % |

| Beispiel M47 | | | |
|---|---|---|---|
| CCH-34 | 6.00 % | Klärpunkt [°C] | +82.5 |
| CCH-501 | 6.00 % | Δn [589 nm, 20 °C] | +0.0662 |
| CCP-2F.F.F | 11.00 % | d · Δn [20 °C] [µm] | 0.50 |
| CCP-3F.F.F | 11.00 % | Verdrillung [°] | 90 |
| CCP-5F.F.F | 6.00 % | V₁₀ [V] | 1.37 |
| CCP-20CF3.F | 6.00 % | | |
| CCP-40CF3 | 5.00 % | | |
| CDU-2-F | 12.00 % | | |
| CDU-3-F | 15.00 % | | |
| CDU-5-F | 8.00 % | | |
| CCOC-3-3 | 8.00 % | | |
| CCOC-3-5 | 6.00 % | | |

| Beispiel M48 | | | |
|---|---|---|---|
| CCH-34 | 6.00 % | Klärpunkt [°C] | +79.0 |
| CCH-501 | 8.00 % | Δn [589 nm, 20 °C] | +0.0656 |
| CCP-2F.F.F | 11.00 % | d · Δn [20 °C] [µm] | 0.50 |
| CCP-3F.F.F | 11.00 % | Verdrillung [°] | 90 |
| CCP-5F.F.F | 6.00 % | V₁₀ [V] | 1.34 |
| CCP-20CF3.F | 4.00 % | | |
| CCP-40CF3 | 6.00 % | | |
| CDU-2-F | 12.00 % | | |
| CDU-3-F | 16.00 % | | |
| CDU-5-F | 8.00 % | | |
| CCOC-3-3 | 8.00 % | | |
| CCOC-3-5 | 4.00 % | | |

| Beispiel M49 | | | |
|---|---|---|---|
| CCH-34 | 6.00 % | S→N [°C] | < -40 |
| CCH-501 | 10.00 % | Klärpunkt [°C] | +80.0 |
| CCH-5CF3 | 6.00 % | Δn [589 nm, 20 °C] | +0.0653 |
| CCP-2F.F.F | 11.00 % | Δε [1kHz, 20 °C] | +9.9 |
| CCP-3F.F.F | 11.00 % | d · Δn [20 °C] [µm] | 0.50 |
| CCP-5F.F.F | 6.00 % | Verdrillung [°] | 90 |
| CCP-20CF3.F | 8.00 % | V₁₀ [V] | 1.33 |
| CCZU-2-F | 6.00 % | | |
| CCZU-3-F | 14.00 % | | |
| CCZU-5-F | 6.00 % | | |
| DCZG-2-OT | 4.00 % | | |
| DCZG-3-OT | 4.00 % | | |
| DCZG-5-OT | 4.00 % | | |
| CCOC-3-3 | 4.00 % | | |

| Beispiel M50 | | | |
|---|---|---|---|
| CCH-35 | 3.00 % | Klärpunkt [°C] | +82.0 |
| CC-5-V | 8.00 % | Δn [589 nm, 20 °C] | +0.0653 |
| CCH-3CF3 | 6.00 % | d · Δn [20 °C] [µm] | 0.55 |
| CCH-5CF3 | 8.00 % | Verdrillung [°] | 90 |
| CCP-2F.F.F | 10.00 % | V₁₀ [V] | 1.54 |
| CCP-3F.F.F | 12.00 % | | |
| CCP-5F.F.F | 4.00 % | | |
| CCZU-2-F | 4.00 % | | |
| CCZU-3-F | 14.00 % | | |
| CCZU-5-F | 4.00 % | | |
| CCP-20CF3.F | 9.00 % | | |
| CCP-30CF3.F | 5.00 % | | |
| CCP-20CF3 | 3.00 % | | |
| CCP-40CF3 | 3.00 % | | |
| CCOC-4-3 | 3.00 % | | |
| CCOC-3-3 | 4.00 % | | |

| Beispiel M51 | | | |
|---|---|---|---|
| CCH-301 | 19.00 % | Klärpunkt [°C] | +95.0 |
| CCH-501 | 17.00 % | Δn [589 nm, 20 °C] | +0.0607 |
| CC-5-V | 3.00 % | d · Δn [20 °C] [um] | 0.55 |
| CCP-2F.F.F | 9.00 % | Verdrillung [°] | 90 |
| CCP-3F.F.F | 7.50 % | V₁₀ [V] | 2.10 |
| CCP-5F.F.F | 3.00 % | | |
| CCZU-2-F | 4.00 % | | |
| CCZU-3-F | 13.00 % | | |
| CCZU-5-F | 4.00 % | | |
| CCPC-33 | 2.00 % | | |
| CCPC-34 | 4.00 % | | |
| CCPC-35 | 4.00 % | | |
| CCOC-3-3 | 3.00 % | | |
| CCOC-4-3 | 4.50 % | | |
| CCOC-3-5 | 3.00 % | | |

| Beispiel M52 | | | |
|---|---|---|---|
| CCH-301 | 13.00 % | Klärpunkt [°C] | +105.8 |
| CCH-501 | 16.00 % | Δn [589 nm, 20 °C] | +0.0594 |
| CCH-34 | 5.00 % | Δε [1 kHz, 20 °C] | +4.7 |
| CCP-2F.F.F | 10.00 % | d · Δn [20 °C] [µm] | 0.50 |
| CCP-3F.F.F | 4.00 % | Verdrillung [°] | 90 |
| CCP-5F.F.F | 4.00 % | V_{10,0,20} [V] | 2.18 |
| CCZU-2-F | 5.00 % | | |
| CCZU-3-F | 15.00 % | | |
| CCZU-5-F | 5.00 % | | |
| CCPC-34 | 2.00 % | | |
| CCPC-35 | 2.00 % | | |
| CCOC-3-3 | 3.00 % | | |
| CCOC-4-3 | 4.00 % | | |
| CCOC-3-5 | 2.00 % | | |
| CCOCC-2-3 | 3.00 % | | |
| CCOCC-3-2 | 4.00 % | | |
| CCOCC-2-2 | 3.00 % | | |

| Beispiel M53 | | | |
|---|---|---|---|
| CCH-301 | 14.00 % | S→N [°C] | < -40 |
| CCH-501 | 16.00 % | Klärpunkt [°C] | +105.5 |
| CCH-34 | 4.00 % | Δn [589,3; nm, 20 °C] | +0.0615 |
| CCP-2F.F.F | 10.00 % | Δε [1 kHz, 20 °C] | +4.7 |
| CCP-3F.F.F | 8.00 % | d · Δn [µm, 20 °C] | 0.50 |
| CCP-5F.F.F | 2.00 % | Verdrillung [°] | 90 |
| CCZU-2-F | 4.00 % | V_{10,0,20}[V] | 2.15 |
| CCZU-3-F | 15.00 % | | |
| CCZU-5-F | 4.00 % | | |
| CCPC-34 | 3.00 % | | |
| CCPC-35 | 3.00 % | | |
| CCOC-3-3 | 3.00 % | | |
| CCOC-4-3 | 4.00 % | | |
| CCOC-3-5 | 2.00 % | | |
| CCOCC-2-3 | 3.00 % | | |
| CCOCC-3-2 | 3.00 % | | |
| CCOCC-2-2 | 2.00 % | | |

| Beispiel M54 | | | |
|---|---|---|---|
| CCH-301 | 15,00 % | S → N [°C] | < -40 |
| CCH-501 | 16.00 % | Klärpunkt [°C] | +104.5 |
| CCH-34 | 4.00 % | Δn [589 nm, 20 °C] | +0.0616 |
| CCP-2F.F.F | 9.00 % | d · Δn [µm, 20 °C] | 0.5 |
| CCP-3F.F.F | 5.00 % | Verdrillung [°] | 90 |
| CCP-5F.F.F | 5.00 % | V_{10,0,20} [V] | 2.17 |
| CCZU-2-F | 4.00 % | | |
| CCZU-3-F | 15.00 % | | |
| CCZU-5-F | 5.00 % | | |
| CCPC-33 | 2.00 % | | |
| CCPC-34 | 3.00 % | | |
| CCPC-35 | 2.00 % | | |
| CCOC-3-3 | 3.00 % | | |
| CCOC-4-3 | 4.00 % | | |
| CCOC-3-5 | 2.00 % | | |
| CCOCC-2-3 | 2.00 % | | |
| CCOCC-3-2 | 2.00 % | | |
| CCOCC-4-3 | 2.00 % | | |

| Beispiel M55 | | | |
|---|---|---|---|
| CCH-301 | 14.00 % | S → N [°C] | < -40 |
| CCH-501 | 14.00 % | Klärpunkt [°C] | +104.5 |
| CCH-34 | 4.00 % | Δn [589 nm, 20 °C] | +0.0618 |
| CCP-2F.F.F | 10.00 % | d · Δn [µm, 20 °C] | 0.5 |
| CCP-3F.F.F | 8.00 % | Verdrillung [°] | 90 |
| CCP-5F.F.F | 4.00 % | V_{10,0,20}[V]: | 2.13 |
| CCZU-2-F | 4.00 % | | |
| CCZU-3-F | 14.00 % | | |
| CCZU-5-F | 4.00 % | | |
| CCPC-33 | 2.00 % | | |
| CCPC-34 | 2.00 % | | |
| CCPC-35 | 2.00 % | | |
| CCOC-3-3 | 3.00 % | | |
| CCOC-4-3 | 4.00 % | | |
| CCOC-3-5 | 2.00 % | | |
| CH-43 | 2.00 % | | |
| CH-45 | 3.00 % | | |
| CCOCC-3-2 | 2.00 % | | |
| CCOCC-2-3 | 2.00 % | | |

| Beispiel M56 | | | |
|---|---|---|---|
| CCH-301 | 18.50 % | Klärpunkt [°C] | +100.0 |
| CCH-501 | 14.00 % | Δn [589 nm, 20 °C] | +0.0608 |
| CC-5-V | 3.00 % | d · Δn [µm, 20 °C] | 0.55 |
| CCP-2F.F.F | 9.00 % | Verdrillung [°] | 90 |
| CCP-3F.F.F | 7.00 % | V_{10,0,20} [V]: | 2.17 |
| CCP-5F.F.F | 3.00 % | | |
| CCZU-2-F | 4.00 % | | |
| CCZU-3-F | 13.00 % | | |
| CCZU-5-F | 4.00 % | | |
| CCPC-33 | 2.00 % | | |
| CCPC-34 | 3.00 % | | |
| CCPC-35 | 3.00 % | | |
| CCOC-3-3 | 3.00 % | | |
| CCOC-4-3 | 4.00 % | | |
| CCOC-3-5 | 3.00 % | | |
| CH-35 | 2.50 % | | |
| CH-43 | 2.00 % | | |
| CH-45 | 2.00 % | | |

| Beispiel M56 | | | |
|---|---|---|---|
| CCH-301 | 22.00 % | Klärpunkt [°C] | +105.0 |
| CCH-501 | 13.00 % | Δn [589 nm, 20 °C] | +0.0612 |
| CCP-2F.F.F | 5.60 % | d · Δn [µm, 20 °C] | 0.55 |
| CCP-3F.F.F | 7.50 % | Verdrillung [°] | 90 |
| CCP-5F.F.F | 4.00% | V_{10,0,20} [V] | 2.21 |
| CCZU-2-F | 3.80 % | | |
| CCZU-3-F | 12.50 % | | |
| CCZU-5-F | 4.60 % | | |
| CCPC-33 | 2.50 % | | |
| CCPC-34 | 2.90 % | | |
| CCPC-35 | 3.10 % | | |
| CCOC-3-3 | 2.85 % | | |
| CCOC-4-3 | 4.45 % | | |
| CCOC-3-5 | 2.20 % | | |
| CH-33 | 1.00 % | | |
| CH-35 | 1.50 % | | |
| CH-43 | 2.00 % | | |
| CH-45 | 2.50 % | | |
| CCOCC-3-2 | 1.00 % | | |
| CCOCC-2-3 | 1.00 % | | |

## Patentansprüche

1. Flüssigkristallines Medium auf der Basis eines Gemisches von polaren Verbindungen mit positiver dielektrischer Anisotropie, **dadurch gekennzeichnet, daß** es eine oder mehrere Verbindungen der allgemeinen Formel IA 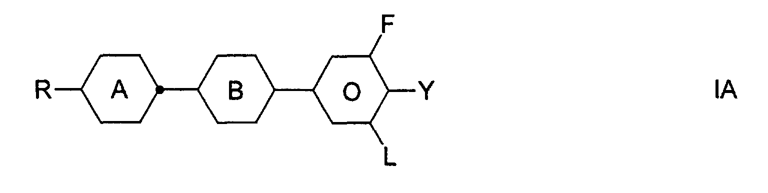 und ein oder mehrere Verbindungen der Formel IB 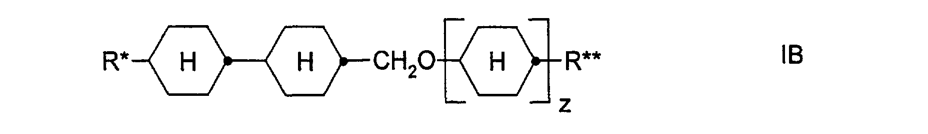 enthält,
worin
R, R* und R** jeweils unabhängig voneinander einen unsubstituierten, einen einfach durch CN oder CF₃ oder einen mindestens einfach durch Halogen substituierten Alkyl- oder Alkenylrest mit 1 bis 12 C-Atomen, wobei in diesen Resten auch eine oder mehrere CH₂-Gruppen jeweils unabhängig voneinander durch -O-, -S-, 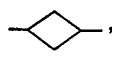 -CO-, -CO-O-, -O-COoder -O-CO-O- so ersetzt sein können, daß O-Atome nicht direkt miteinander verknüpft sind und 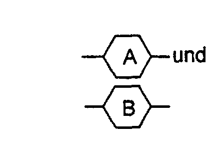 jeweils unabhängig voneinander 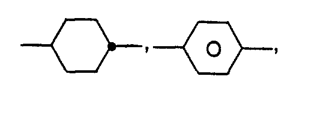 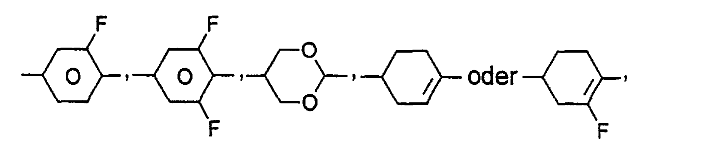
L H oder F, und
Y F, Cl, CN, halogeniertes Alkyl, halogeniertes Alkenyloxy, halogeniertes Alkoxy oder halogeniertes Alkenyl mit bis zu 9 C-Atomen
z 1 oder 2,
bedeuten.

2. Medium nach Anspruch 1, **dadurch gekennzeichnet, daß** es zusätzlich eine oder mehrere Verbindungen ausgewählt aus der Gruppe bestehend aus den allgemeinen Formeln II, III, IV, V, VI, Vll und VIII enthält: 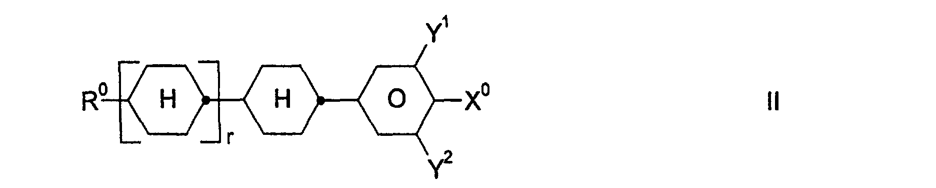 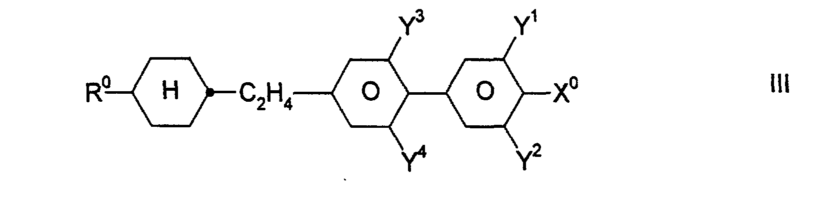 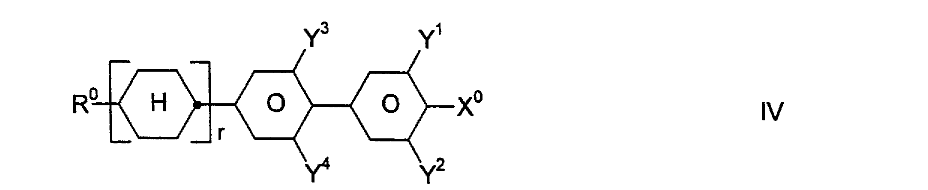 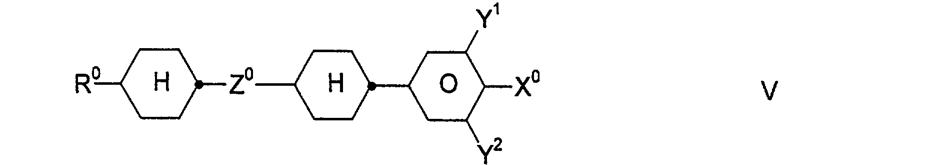 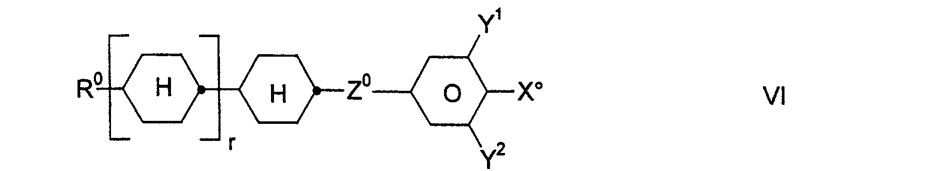 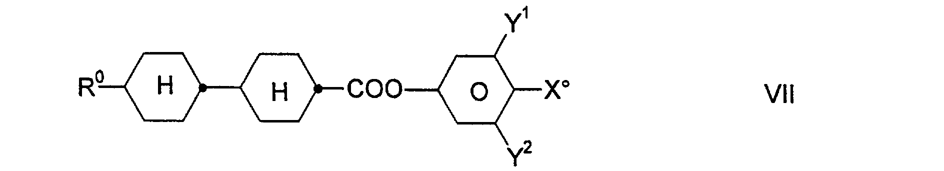 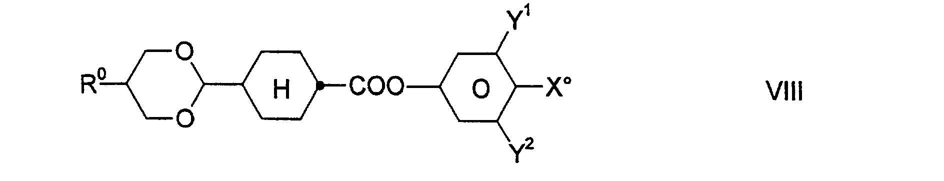 worin die einzelnen Reste die folgenden Bedeutungen haben:
R⁰: n-Alkyl, Oxaalkyl, Fluoralkyl, Alkenyloxy oder Alkenyl mit jeweils bis zu 9 C-Atomen,
X⁰: F, Cl, halogeniertes Alkyl, Alkenyl oder Alkoxy mit 1 bis 6 C-Atomen,
Z⁰: -C₂H₄-,-CF=CF-, -CH₂O-, -OCH₂-, -CF₂O-, -OCF₂- oder -C₄F₄-,
Y¹ bis Y⁴: jeweils unabhängig voneinander H oder F,
r: 0 oder 1,
wobei die Verbindungen der Formeln II und IV mit den Verbindungen der Formel IA nicht identisch sind.

3. Medium nach Anspruch 2, **dadurch gekennzeichnet, daß** der Anteil an Verbindungen der Formeln IA und IB zusammen im Gesamtgemisch mindestens 10 bis 100 Gew.-% beträgt.

4. Medium nach Anspruch 1 oder 2, **dadurch gekennzeichnet, daß** der Anteil an Verbindungen der Formel II bis VIII im Gesamtgemisch 10 bis 50 Gew.-% beträgt.

5. Medium nach mindestens einem der Ansprüche 1 bis 4, **dadurch gekennzeichnet, daß** die Mischung zwei oder drei Verbindungen der Formel IB enthält.

6. Medium nach Anspruch 1, **dadurch gekennzeichnet, daß** es zusätzlich eine oder mehrere Verbindungen der Formeln RI bis RV 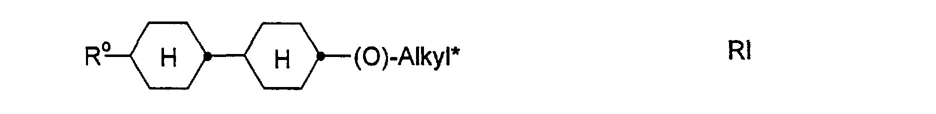 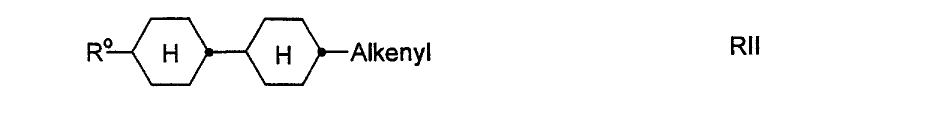 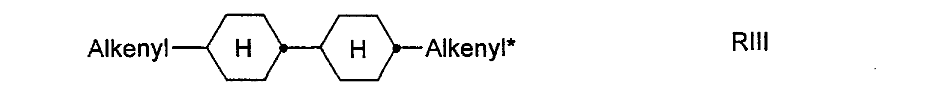 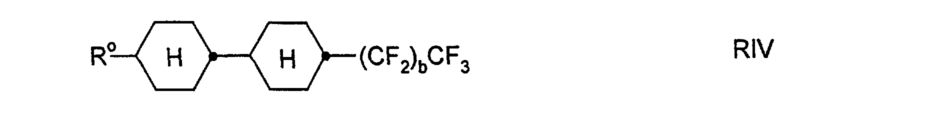 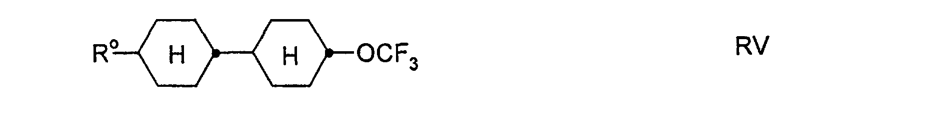 enthält,
worin
R⁰ n-Alkyl, Oxaalkyl, Fluoralkyl, Alkenyloxy oder Alkenyl mit jeweils bis zu 9 C-Atomen,
b: 0, 1 oder 2
Alkyl und Alkyl*: jeweils unabhängig voneinander geradkettige oder verzweigte Alkylgruppen mit 1 bis 9 Kohlenstoffatomen, und
Alkenyl und Alkenyl*: jeweils unabhängig voneinander geradkettige oder verzweigte Alkenylgruppen mit 2 bis 9 Kohlenstoffatomen
bedeuten.

7. Medium nach einem der Ansprüche 2 bis 6, **dadurch gekennzeichnet, daß** X⁰ F oder OCF₃ und Y² H oder F bedeuten.

8. Medium nach einem der Ansprüche 1 bis 7, **dadurch gekennzeichnet, daß** in der Verbindung der Formel IA L = F ist.

9. Medium nach einem der Ansprüche 1 bis 8, **dadurch gekennzeichnet, daß** Y in den Verbindungen der Formel IA
F, Cl, CN, CF₃, CF₂H, OCF₃, OCF₂H, OCFHCF₃, OCFHCFH₂, OCFHCF₂H, OCF₂CH₃, OCF₂CFH₂, OCF₂CF₂H, OCF₂CF₂CF₂H, OCF₂CF₂CFH₂, OCFHCF₂CF₃, OCFHCF₂CF₂H, OCF₂CF₂CF₃, OCF₂CHFCF₃, OCClFCF₂CF₃ oder OCH = CF₂.
bedeutet.

10. Verbindungen der Formel IB 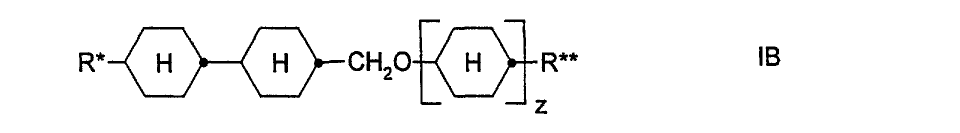 worin
R* und R** jeweils unabhängig voneinander einen unsubstituierten, einen einfach durch CN oder CF₃ oder einen mindestens einfach durch Halogen substituierten Alkyl- oder Alkenylrest mit 1 bis 12 C-Atomen, wobei in diesen Resten auch eine oder mehrere CH₂-Gruppen jeweils unabhängig voneinander durch -O-, -S-, 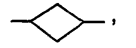 -CO-, -CO-O-, -O-COoder -O-CO-O- so ersetzt sein können, daß O-Atome nicht direkt miteinander verknüpft sind, und
z 1 oder 2
bedeuten.

11. Verwendung des flüssigkristallinen Mediums nach Anspruch 1 für elektrooptische Zwecke.

12. Elektrooptische Flüssigkristallanzeige enthaltend ein flüssigkristallines Medium nach Anspruch 1.

## Claims

1. Liquid-crystalline medium on the basis of a mixture of polar compounds having positive dielectric anisotropy, **characterized in that** it comprises one or more compounds of the general formula IA 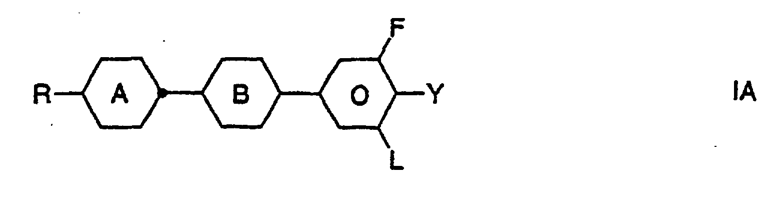 and one or more compounds of the formula IB 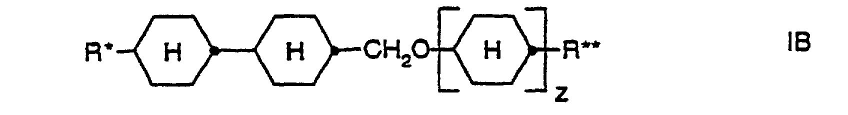 where
R, R* and R** each, independently of one another, are an alkyl or alkenyl radical having from 1 to 12 C atoms which is unsubstituted, singly substituted by CN or CF₃ or at least singly substituted by halogen, with the additional option of one or more CH₂ groups in these radicals, independently of one another in each case, being replaced by -O-, -S-, 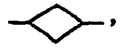 -CO-, -CO-O-, -O-CO- or -O-CO-O- in such a way that O atoms are not directly linked together, and 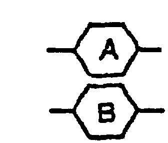 and each, independently of one another, are 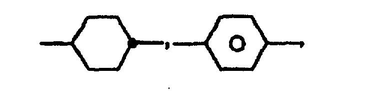 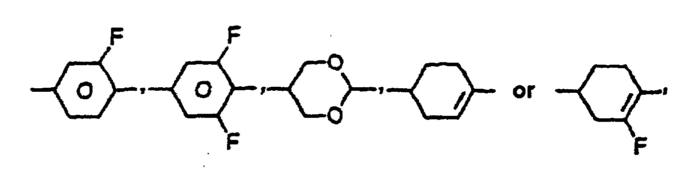
L is H or F, and
Y is F, Cl, CN, halogenated alkyl, halogenated alkenyloxy, halogenated alkoxy or halogenated alkenyl having up to 9 C atoms, and
z is 1 or 2.

2. Medium according to Claim 1, **characterized in that** it additionally comprises one or more compounds selected from the group consisting of the general formulae II, III, IV, V, VI, VII and VIII: 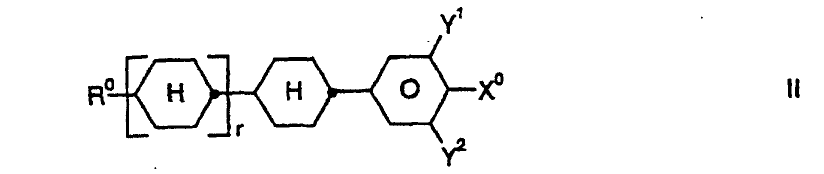 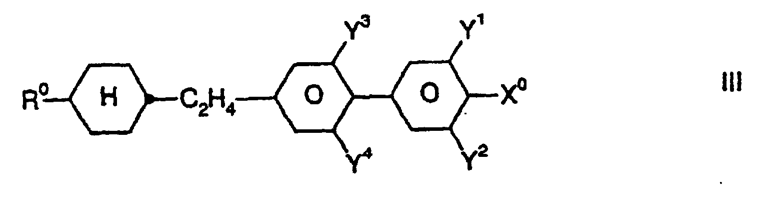 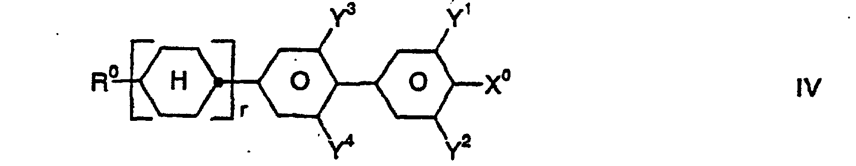 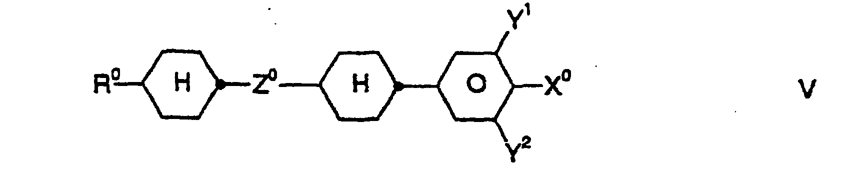 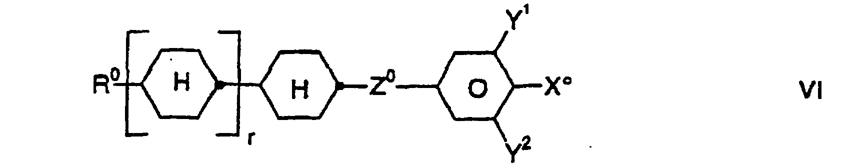 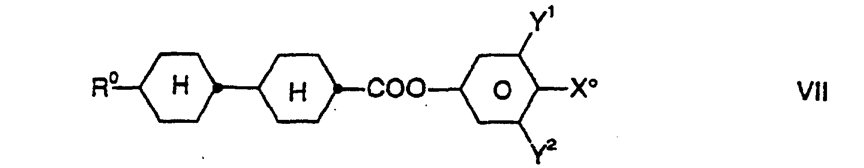 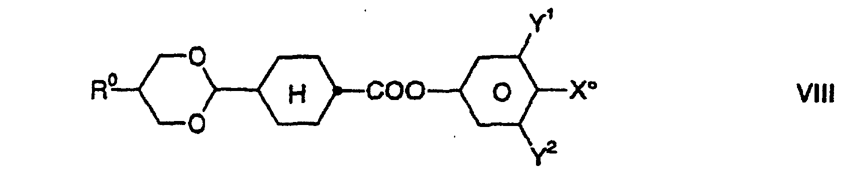 where the individual radicals have the following meanings:
R⁰: n-alkyl, oxaalkyl, fluoroalkyl, alkenyloxy or alkenyl each having up to 9 C atoms,
X⁰: F, Cl, halogenated alkyl, alkenyl or alkoxy having from 1 to 6 C atoms,
Z⁰: -C₂H₄-, -CF=CF-, -CH₂O-, -OCH₂-, -CF₂O-, -OCF₂- or -C₄F₄-,
Y¹ to Y⁴: each, independently of one another, H or F,
r: 0 or 1,
the compounds of the formulae II and IV not being identical with the compounds of the formula IA.

3. Medium according to Claim 2, **characterized in that** the proportion of compounds of the formulae IA and IB jointly in the mixture as a whole is at least from 10 to 100 wt%.

4. Medium according to Claim 1 or 2, **characterized in that** the proportion of compounds of the formulae II to VIII in the mixture as a whole is from 10 to 50 wt%.

5. Medium according to at least one of Claims 1 to 4, **characterized in that** the mixture comprises two or three compounds of the formula IB.

6. Medium according to Claim 1, **characterized in that** it additionally comprises one or more compounds of the formulae RI to RV 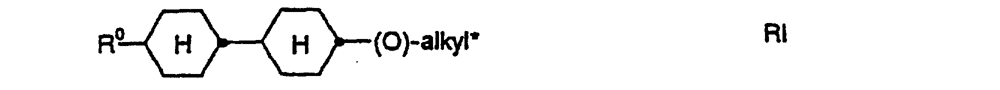 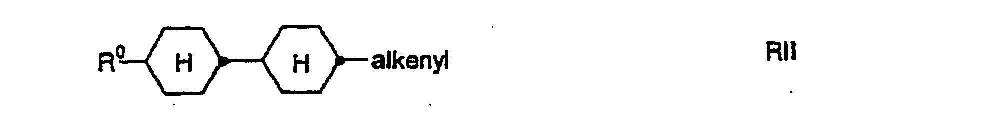 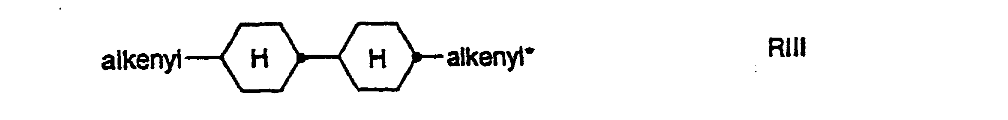 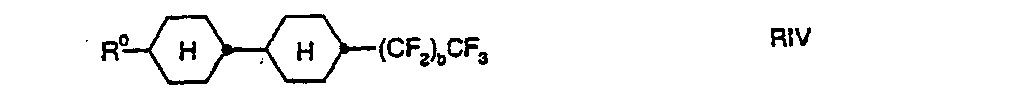 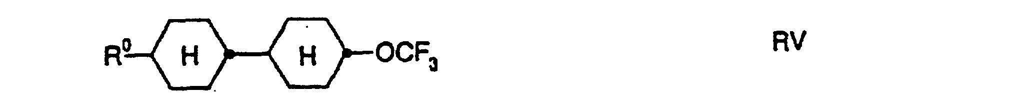 where
R⁰: n-alkyl, oxaalkyl, fluoroalkyl, alkenyloxy or alkenyl each having up to 9 C atoms,
b: 0, 1 or 2,
alkyl and alkyl*: each, independently of one another, straight-chain or branched alkyl groups having from 1 to 9 carbon atoms, and
alkenyl and alkenyl*: each, independently of one another, straight-chain or branched alkenyl groups having from 2 to 9 carbon atoms.

7. Medium according to any one of Claims 2 to 6, **characterized in that** X⁰ is F or OCF₃, and Y² is H or F.

8. Medium according to any one of Claims 1 to 7, **characterized in that** in the compound of the formula IA, L = F.

9. Medium according to any one of Claims 1 to 8, **characterized in that** Y in the compounds of the formula IA is
F, Cl, CN, CF₃, CF₂H, OCF₃, OCF₂H, OCFHCF₃, OCFHCFH₂, OCFHCF₂H, OCF₂CH₃, OCF₂CFH₂, OCF₂CF₂H, OCF₂CF₂CF₂H, OCF₂CF₂CFH₂, OCFHCF₂CF₃, OCFHCF₂CF₂H, OCF₂CF₂CF₃, OCF₂CHFCF₃, OCCIFCF₂CF₃ or OCH = CF₂.

10. Compounds of the formula IB 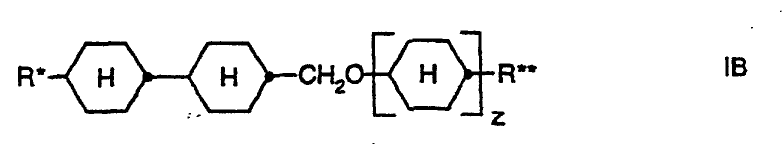 where
R* and R** each, independently of one another, are an alkyl or alkenyl radical having from 1 to 12 C atoms which is unsubstituted, singly substituted by CN or CF₃ or at least singly substituted by halogen, with the additional option of one or more CH₂ groups in these radicals, independently of one another in each case, being replaced by -O-, -S-, 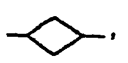 -CO-, -CO-O-, -O-CO- or -O-CO-O-, in such a way that O atoms are not directly linked together, and
z is 1 or 2.

11. Use of the liquid-crystalline medium according to Claim 1 for electro-optical purposes.

12. Electro-optical liquid crystal display comprising a liquid-crystalline medium according to Claim 1.

## Revendications

1. Agent liquide cristallin sur base d'un mélange de composés polaires avec anisotropie diélectrique positive, **caractérisé en ce qu'**il comprend un ou plusieurs composés de la formule générale lA 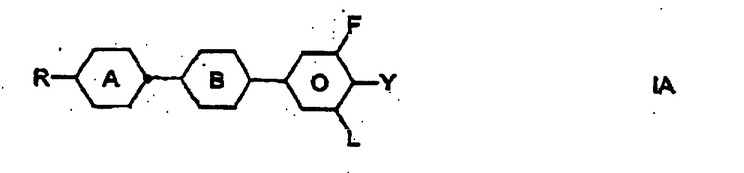 et un ou plusieurs composés de la formule IB 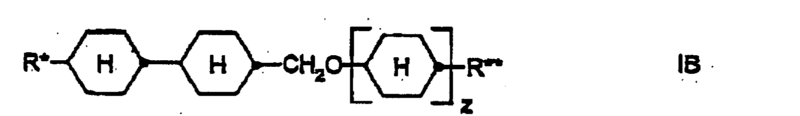 dans lesquelles
R, R* et R** sont indépendamment l'un de l'autre un reste alkyle ou alcényle avec 1 à 12 atomes de C, non substitué, substitué simplement par CN ou CF₃ ou au minimum simplement substitué par un halogène, un ou plusieurs groupes CH₂ dans ces restes pouvant être remplacés indépendamment par -O-,-S-, 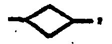 -CO-,-CO-O-,-O-CO- ou par -O-CO-O, de sorte que les atomes O ne soient pas directement reliés entre eux et 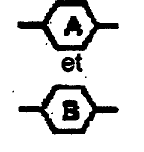 représentent indépendamment l'un de l'autre : 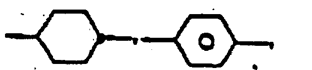 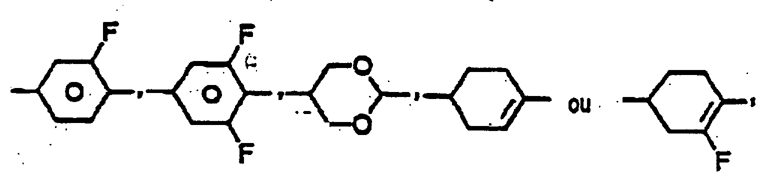
L représente H ou F, et
Y représente F, Cl, CN, un alkyle Halogéné, un alcényloxy halogène, un alcoxy halogène ou un alcényle halogéné avec 9 atomes de C ou moins
Z est 1 ou 2.

2. Agent selon la revendication 1, **caractérisé en ce qu'**il contient par ailleurs un ou plusieurs composés sélectionnés parmi le groupe se composant des formules générales II, III, IV, V, VI, VII et VIII : 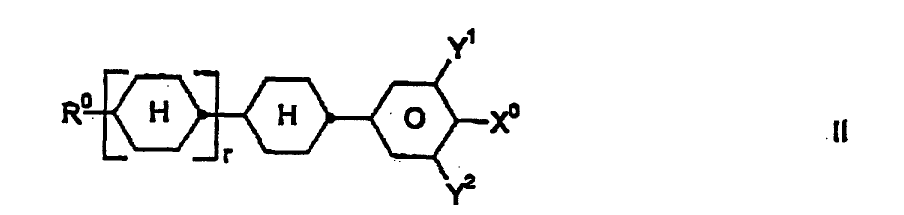 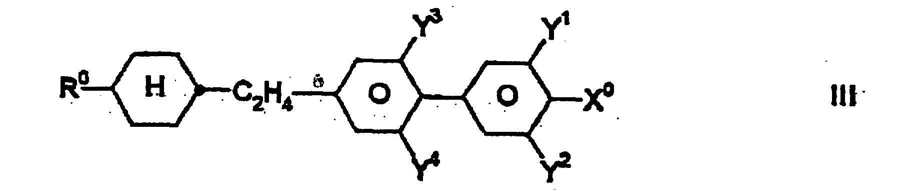 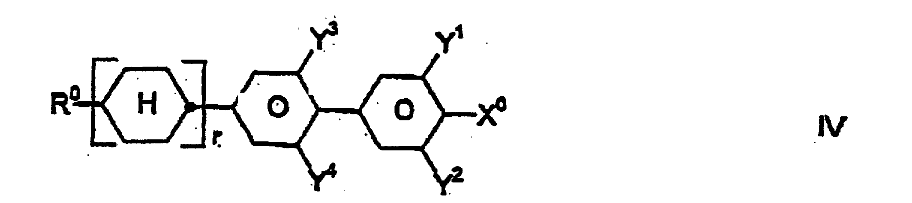 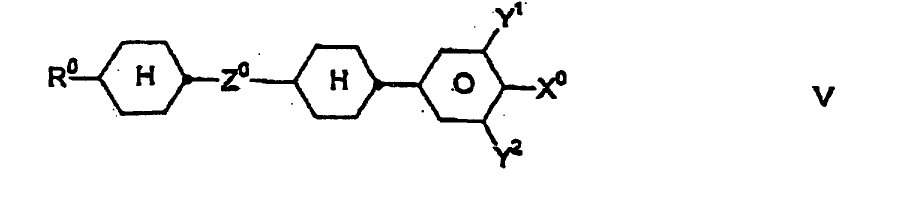 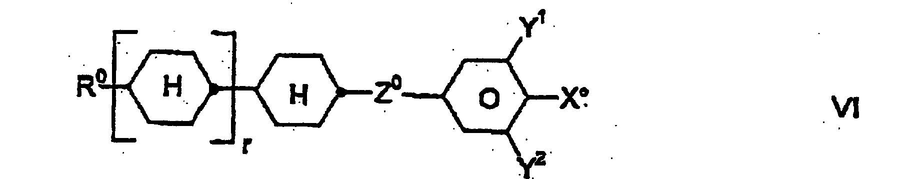 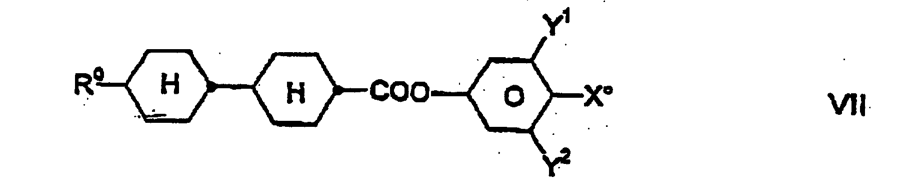 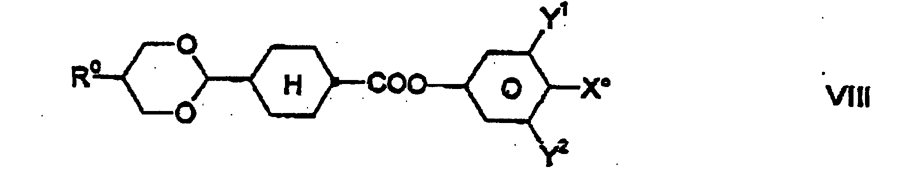 dans lesquelles les différents restes ont les significations suivantes :
R⁰ n-alkyle, oxaalkyle, fluoroalkyle, alcényloxy ou alcényle avec chaque fois 9 atomes de C ou moins,
X⁰ F, Cl, alkyle halogène, alcényle ou alcoxy avec 1 à 6 atomes de C,
Z⁰ -C₂H₄-,-CF=CF-,-CH₂O-,-OCH₂-,-CF₂O-,-OCF₂- ou -C₄F₄-,
Y¹ à Y⁴ F ou H chaque fois indépendamment l'un de l'autre,
r 0 ou 1,
les composés des formules II et IV n'étant pas identiques avec les composés de formule IA,

3. Agent selon la revendication 2, **caractérisé en ce que** la proportion de composés des formules IA et IB dans leur ensemble dans le mélange total est au minimum de 10 à 100 % en poids.

4. Agent selon l'une quelconque des revendications 1 ou 2, **caractérisé en ce que** la proportion de composés de formules II à VIII dans le mélange total est de 10 à 50 % en poids.

5. Agent selon au moins l'une quelconque des revendications 1 à 4, **caractérisé en ce que** le mélange comprend deux ou trois composés de formule IB.

6. Agent selon la revendication 1, **caractérisé en ce qu'**il comprend par ailleurs un ou plusieurs composés des formules RI à RV 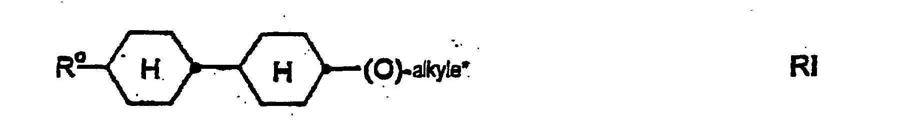 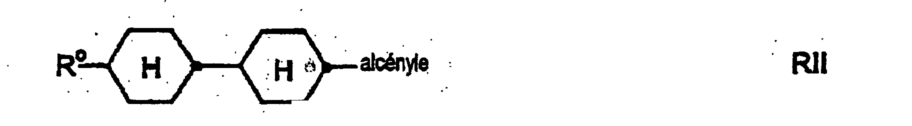 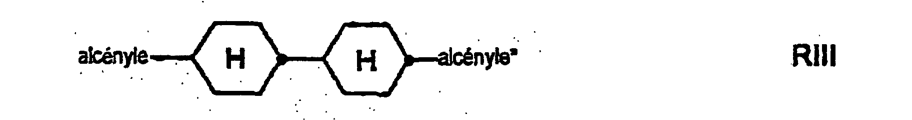 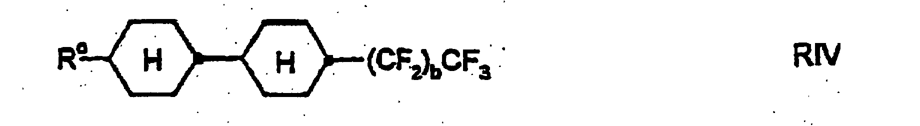 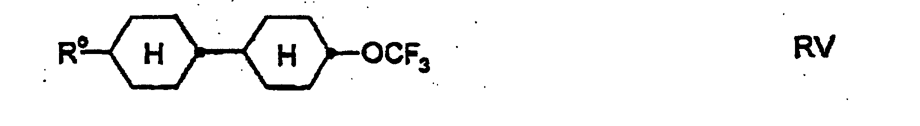 dans lesquelles on a les significations suivantes :
R⁰ n-alkyle, oxaalkyle, fluoroalkyle, alcényloxy ou alcényle avec chaque fois 9 atomes de C ou moins,
b 0,1 ou 2
alkyle et alkyle*: indépendamment l'un de l'autre, groupes alkyles à chaînes droites ou ramifiées, avec 1 à 9 atomes de carbone, et
alcényle et alcényle*: indépendamment l'un de l'autre, groupes alcényles à chaînes droites ou ramifiées, avec 2 à 9 atomes de carbone.

7. Agent selon l'une quelconque des revendications 2 à 6 **caractérisé en ce que** X⁰ signifie F ou OCF₃ et Y², H ou F.

8. Agent selon l'une quelconque des revendications 1 à 7 **caractérisé en ce que** dans le composé de la formule IA, L = F.

9. Agent selon l'une quelconque des revendications 1 à 8 **caractérisé en ce que** dans le composé de la formule IA
Y signifie
F, Cl, CN, CF₃, CF₂H, OCF₃, OCF₂H, OCFHCF₃, OCFHCFH₂, OCFHCF₂H, OCF₂CH₃, OCF₂CFH₂, OCF₂CF₂H, OCF₂CF₂CF₂H, OCF₂CF₂CFH₂, OCFHCF₂CF₃, OCFHCF₂CF₂H, OCF₂CF₂CF₃, OCF₂CHFCF₃, OCCIFCF₂CF₃ ou OCH = CF₂.

10. Composés de formule IB 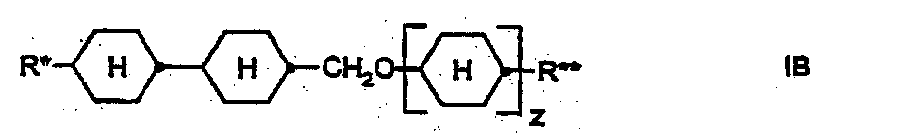 dans lesquelles
R* et R** sont indépendamment l'un de l'autre un reste alkyle ou alcényle avec 1 à 12 atomes de C non substitué, substitué simplement par CN ou CF₃ ou au minimum simplement substitué par un halogène, un ou plusieurs groupes CH₂ dans ces restes pouvant être remplacés indépendamment l'un de l'autre par -O-, -S-, 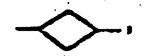 -CO-, -CO-O-, -O-CO- ou par -O-CO-O, de sorte que les atomes O ne soient pas directement reliés entre eux et
z signifie 1 ou 2

11. Utilisation de l'agent liquide cristallin selon la revendication 1, à des fins électro-optiques.

12. Affichage électro-optique à cristaux liquides comprenant un agent liquide cristallin selon la revendication 1.
